# EUROPEAN PATENT APPLICATION

(11) **EP 4 458 349 A1**
(43) Date of publication of application: **06.11.2024**
(21) Application number: 22915868.8
(22) Date of filing: 21.12.2022
(51) Int. Cl.: A61K 9/127, A61K 35/12, A61K 47/69, A61P 11/00, A61P 43/00, C12N 5/0735, C12N 5/0775, C12N 5/10, C12N 15/09

(54) **EXOSOME, METHOD FOR FORMING SAME, AND COMPOSITION CONTAINING SAME**

(30) Priority: 28.12.2021 JP 2021214849; 28.12.2021 JP 2021214878; 28.12.2021 JP 2021214889; 20.07.2022 JP 2022115931
(71) Applicant: SEKISUI CHEMICAL CO., LTD., Osaka-shi Osaka 530-8565 (JP)
(72) Inventor: TAN Zheli, Tsukuba-shi, Ibaraki 300-4247 (JP)
(74) Representative: Ter Meer Steinmeister & Partner
(86) International application number: PCT/JP2022/047115
(87) International publication number: WO 2023/127645

(57) **Abstract**

An exosome comprising at least one exosome marker protein selected from among CD63, CD81 and CD9, and an integrin α and/or an integrin β in a lipid bilayer is disclosed. This exosome allows drug delivery with high target selectivity.

## Description

### [Cross-reference to Related Applications]

Priority is claimed on Japanese Patent Application No. 2022-115931, filed July 20, 2022, Japanese Patent Application No. 2021-214889, filed December 28, 2021, Japanese Patent Application No. 2021-214878, filed December 28, 2021, and Japanese Patent Application No. 2021-214849, filed December 28, 2021, the entire content of which is incorporated as a part of this specification by reference.

### [Technical Field]

The present invention relates to an exosome into which an integrin α and/or an integrin β has been introduced, and a method of forming an exosome, the method including a step of overexpressing an integrin α and/or an integrin β.

### [Background Art]

It is generally known that, when a drug having pharmacological effects is administered to a human body, it inevitably causes undesirable side effects in addition to intended medicinal effects of the drug. In order to reduce side effects of such drugs and increase the efficacy, in recent years, a drug delivery system (DDS) has been proposed. When the drug delivery system is used, since the drug of interest is selectively delivered to the lesion, the efficacy can be exhibited with a lower dosage, and a possibility of the drug being delivered to body parts other than the lesion and undesired side effects being caused can be reduced. Here, regarding application of nanostructures using organic polymers to the drug delivery system, how to impart a controlled release function (sustained release) and a targeting function (cell tropism/affinity) in the blood to capsules encapsulating drugs (called "carriers") is the key for development.

It has been pointed out that liposomes, which are nanoparticles composed of phospholipids, and the like are highly useful as carriers used in such a drug delivery system. Liposomes encapsulating drugs have an advantage that they can be relatively easily formed by mixing drugs and phospholipids in vitro, and have become widely used components in the field of drug delivery systems. However, since liposomes are reconstructed in vitro, it is difficult to impart a targeting function to target cells on liposome themselves.

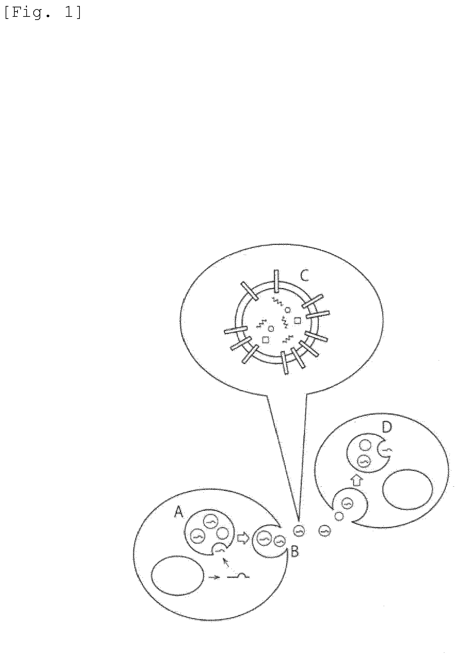

Incidentally, in recent years, it has become known that a type of vesicle called an exosome is released from cells. Fig. 1 is a diagram illustrating an endosome formation and cell incorporation state. Exosomes are lipid bilayer vesicles with a diameter of 20 nm to 150 nm. As shown in Fig. 1, exosomes encapsulate proteins and nucleic acids such as miRNA and mRNA therein, and have proteins on their surfaces. Exosomes are generally formed in multi-vesicular bodies (A in Fig. 1) formed within cells by budding from the cytoplasm toward the lumen of the multi-vesicular bodies, and are released to the outside of the cells by the fusion of the multi-vesicular bodies and the cell membrane (B in Fig. 1). As shown in C in Fig. 1, exosomes contain labeled molecules derived from the origin cells, and have tropism according to characteristics of the origin cells, and are thought to be involved in physiological effects such as blood coagulation and intercellular signal transmission. When such exosomes come into contact with target cells, they are incorporated into the target cells by endocytosis, the lipid bilayers of endosomes and exosomes are fused, and proteins and nucleic acids such as miRNA and mRNA encapsulated in the exosomes are released into the cytoplasm (D in Fig. 1).

Here, an attempt has been made to apply these exosomes to drug delivery systems, and selectively deliver drugs to specific cells and tissues using targeting of exosomes. For example, PTL 1 discloses a hybrid liposome exosome in which a liposome encapsulating a physiologically active substance and an exosome are combined, wherein the exosome is a vesicle released from cells, has a diameter of 30 nm to 200 nm, and contains phospholipids, cholesterol, proteins and nucleic acids. According to the invention described in PTL 1, when the liposome and the exosome are combined, a substance to be encapsulated in the liposome can also be encapsulated in the exosome. In addition, PTL 2 discloses an exosome prepared by an exosome isolation method including a step of providing a sample containing an exosome; a step of bringing this sample into contact with a binder having an affinity for a target protein selected from among PTGFRN, BSG, IGSF2, IGSF3, IGSF8, ITGB1, ITGA4, SLC3A2, and ATP transporters, fragments thereof or mutants thereof; and a step of isolating the exosome based on binding between the target protein and the binder. An object of the invention described in PTL 2 is to improve efficiency of treatments and the like using exosomes by streamlining an exosome isolation and purification method.

### [Citation List]

### [Patent Literature]

[PTL 1] Japanese Patent No. 6137894
[PTL 2] WO 2019/040920

### [Summary of Invention]

However, the inventions disclosed in PTL 1 and 2 relate to an initial study of a drug delivery system using an exosome. The hybrid liposome exosome disclosed in PTL 1 is expected to have relatively improved targeting, but the exosomes disclosed in PTL 1 and 2 have a problem that it is not possible to deliver a drug with sufficient target selectivity for practical use as a carrier for a drug delivery system.

Therefore, the present invention has been made in view of the above circumstances, and an object of the present invention is to provide an exosome that can deliver drugs with high target selectivity and the like.

The inventors of the present invention conducted extensive studies in view of the above circumstances. As a result, they found that an exosome containing at least an integrin α and/or an integrin β in a lipid bilayer can address the above problems. The present invention is based on this finding.

Specifically, the present invention includes the following inventions.
(1) An exosome including at least one exosome marker protein selected from among CD63, CD81 and CD9, and an integrin α and/or an integrin β in a lipid bilayer.
(2) The exosome according to (1), further including a protein disulfide isomerase (PDI) in the lipid bilayer.
(3) The exosome according to (2),
   wherein the exosome marker protein is selected from among CD63, CD81 and CD9, and
   wherein the exosome contains 0.01 ng or more and 100 ng or less of the protein disulfide isomerase (PDI) per 1 ng of the total amount of the exosome marker protein present in the exosome.
(4) The exosome according to (2) or (3),
   wherein the molar ratio of the protein disulfide isomerase (PDI) to the integrin α and the integrin β is 1:0.1 to 1:10.
(5) The exosome according to any one of (2) to (4),
   wherein the protein disulfide isomerase (PDI) is selected from among
   (i) human protein disulfide isomerase A4 including an amino acid sequence shown in SEQ ID No. 6;
   (ii) human protein disulfide isomerase A1 including an amino acid sequence shown in SEQ ID No. 7;
   (iii) human protein disulfide isomerase A3 including an amino acid sequence shown in SEQ ID No. 8;
   (iv) human protein disulfide isomerase A6 including an amino acid sequence shown in SEQ ID No. 9; and
   (v) a mutant protein including an amino acid sequence having at least 90% sequence homology to the amino acid sequence shown by any of SEQ ID Nos. 6 to 9 and functioning as a protein disulfide isomerase.
(6) The exosome according to any one of (1) to (5),
   wherein the exosome marker protein is selected from among CD63, CD81 and CD9, and
   wherein the exosome contains 0.01 ng or more and 100 ng or less of the integrin α per 1 ng of the total amount of the exosome marker protein present in the exosome, or
   0.01 ng or more and 100 or less of the integrin β per 1 ng of the total amount of the exosome marker protein present in the exosome.
(7) The exosome according to any one of (1) to (6),
   wherein the integrin α is selected from among
      (i) human integrin α4 including an amino acid sequence shown in SEQ ID No. 10;
      (ii) human integrin αv including an amino acid sequence shown in SEQ ID No. 11;
      (iii) human integrin α6 including an amino acid sequence shown in SEQ ID No. 12; and
      (iv) a mutant protein including an amino acid sequence having at least 90% sequence homology to the amino acid sequence shown by any of SEQ ID Nos. 10 to 12 and functioning as an integrin α, and
   wherein the integrin β is selected from among
      (v) human integrin β4 including an amino acid sequence shown in SEQ ID No. 13;
      (vi) human integrin β5 including an amino acid sequence shown in SEQ ID No. 14;
      (vii) human integrin β6 including an amino acid sequence shown in SEQ ID No. 15; and
      (viii) a mutant protein including an amino acid sequence having at least 90% sequence homology to the amino acid sequence shown by any of SEQ ID Nos. 13 to 15 and functioning as an integrin β.
(8) A method of forming an exosome, the method including
   an overexpression step in which an integrin α and/or an integrin β is overexpressed in cells; and
   a collection step in which the exosome according to any one of (1) to (7) is collected from the cells in which the integrin α and/or the integrin β is overexpressed.
(9) The method of forming an exosome according to (8),
   wherein the cells are at least one selected from the group consisting of pluripotent stem cells, mesenchymal stem cells, ectoderm-derived cells, mesoderm-derived cells, and endoderm-derived cells.
(10) A method of forming a hybrid liposome exosome, the method including
   a hybrid liposome exosome forming step in which the exosome formed by the method of forming an exosome according to (8) or (9) is fused with a liposome to form a hybrid liposome exosome.
(11) A hybrid liposome exosome formed by the method of forming a hybrid liposome exosome according to (10).
(12) A hybrid liposome exosome including at least one exosome marker protein selected from among CD63, CD81 and CD9, an integrin α, and an integrin β in a lipid bilayer.
(13) The hybrid liposome exosome according to (12), further including a protein disulfide isomerase (PDI) in the lipid bilayer.
(14) The hybrid liposome exosome according to (13),
   wherein the molar ratio of the protein disulfide isomerase (PDI) to the integrin α and the integrin β is 1:0.1 to 1:10.
(15) The hybrid liposome exosome according to (13) or (14),
   wherein the protein disulfide isomerase (PDI) is selected from among
   (i) human protein disulfide isomerase A4 including an amino acid sequence shown in SEQ ID No. 6;
   (ii) human protein disulfide isomerase A1 including an amino acid sequence shown in SEQ ID No. 7;
   (iii) human protein disulfide isomerase A3 including an amino acid sequence shown in SEQ ID No. 8;
   (iv) human protein disulfide isomerase A6 including an amino acid sequence shown in SEQ ID No. 9; and
   (v) a mutant protein including an amino acid sequence having at least 90% sequence homology to the amino acid sequence shown by any of SEQ ID Nos. 6 to 9 and functioning as a protein disulfide isomerase.
(16) The hybrid liposome exosome according to any one of (12) to (15),
   wherein the integrin α is selected from among
      (i) human integrin α4 including an amino acid sequence shown in SEQ ID No. 10;
      (ii) human integrin αv including an amino acid sequence shown in SEQ ID No. 11;
      (iii) human integrin α6 including an amino acid sequence shown in SEQ ID No. 12; and
      (iv) a mutant protein including an amino acid sequence having at least 90% sequence homology to the amino acid sequence shown by any of SEQ ID Nos. 10 to 12 and functioning as an integrin α,
   wherein the integrin β is selected from among
      (v) human integrin β4 including an amino acid sequence shown in SEQ ID No. 13;
      (vi) human integrin β5 including an amino acid sequence shown in SEQ ID No. 14;
      (vii) human integrin β6 including an amino acid sequence shown in SEQ ID No. 15; and
      (viii) a mutant protein including an amino acid sequence having at least 90% sequence homology to the amino acid sequence shown by any of SEQ ID Nos. 13 to 15 and functioning as an integrin β.
(17) A composition including the exosome according to any one of (1) to (7), or the hybrid liposome exosome according to any one of (11) to (16), and a pharmaceutically acceptable carrier.
(18) The composition according to (17), which is used to treat a lung disease, alleviate lung disease symptoms, and/or prevent a lung disease.

The present invention also includes the following inventions.
(A1) An exosome including at least one exosome marker protein selected from among CD63, CD81 and CD9, an integrin α and/or an integrin β and/or a mutant protein having a sequence homology of at least 90% therewith, and a protein disulfide isomerase (PDI) in a lipid bilayer.
(A2) The exosome according to (A1),
   wherein the exosome marker protein is selected from among CD63, CD81 and CD9, and
   wherein the exosome contains 0.01 ng or more and 100 ng or less of the protein disulfide isomerase (PDI) per 1 ng of the total amount of the exosome marker protein present in the exosome.
(A3) The exosome according to (A1) or (A2),
   wherein the exosome marker protein is selected from among CD63, CD81 and CD9, and
   wherein the exosome includes 0.01 ng or more and 100 ng or less of the integrin α, and/or a mutant protein having a sequence homology of at least 90% therewith per 1 ng of the total amount of the exosome marker protein present in the exosome, or
   0.01 ng or more and 100 or less of the integrin β, and/or a mutant protein having a sequence homology of at least 90% therewith per 1 ng of the total amount of the exosome marker protein present in the exosome.
(A4) The exosome according to any one of (A1) to (A3),
   wherein the integrin α is selected from among an integrin α4, an integrin 5, and an integrin α6, and
   wherein the integrin β is selected from among an integrin β4, an integrin β5, and an integrin β6.
(A5) A method of forming an exosome, the method including:
   an overexpression step in which an integrin α and/or an integrin β, and/or a mutant protein having a sequence homology of at least 90% therewith are overexpressed in cells; and
   a collection step in which an exosome is collected from the cells in which the integrin α and/or the integrin β, and/or the mutant protein having a sequence homology of at least 90% therewith are overexpressed.
(A6) The method of forming an exosome according to (A5),
   wherein the exosome collected in the collection step includes at least one exosome marker protein selected from among CD63, CD81 and CD9, and
   wherein the exosome contains 0.01 ng or more and 100 ng or less of the integrin α and/or the mutant protein having a sequence homology of at least 90% therewith per 1 ng of the total amount of the exosome marker protein present in the exosome or 0.01 ng or more and 100 ng or less of the integrin β and/or the mutant protein having a sequence homology of at least 90%.
(A7) The method of forming an exosome according to (A5) or (A6),
   wherein the cells are at least one selected from the group consisting of pluripotent stem cells, mesenchymal stem cells, ectoderm-derived cells, mesoderm-derived cells, and endoderm-derived cells.
(A8) The exosome formed by the method of forming an exosome according to any one of (A5) to (A7).
(A9) An exosome-containing composition including the exosome according to any one of (A1) to (A4) and (A8), and a pharmaceutically acceptable carrier.
(A10) An exosome including a protein disulfide isomerase (PDI) in a lipid bilayer.
(A11) The exosome according to (A10), including 0.01 ng or more and 100 ng or less of the protein disulfide isomerase (PDI) per 1 ng of the total amount of at least one exosome marker protein selected from among CD63, CD81 and CD9 contained in the lipid bilayer.
(A12) The exosome according to (A10), including 0.01 ng or more and 100 ng or less of the protein disulfide isomerase (PDI) per 1 ng of the total amount of phospholipids contained in the lipid bilayer.

The present invention also includes the following inventions.
(B1) A hybrid liposome exosome including at least one exosome marker protein selected from among CD63, CD81 and CD9, an integrin α, and an integrin β in a lipid bilayer.
(B2) The hybrid liposome exosome according to (B1),
wherein the exosome marker protein is selected from among CD63, CD81 and CD9, and
wherein the hybrid liposome exosome contains 0.01 ng or more and 100 ng or less of the integrin α per 1 ng of the total amount of the exosome marker protein present in the exosome, and
0.01 ng or more and 100 ng or less of the integrin β per 1 ng of the total amount of the exosome marker protein present in the exosome.
(B3) The hybrid liposome exosome according to (B1) or (B2),
wherein the integrin α is selected from among an integrin α4, an integrin 5, and an integrin α6, and
wherein the integrin β is selected from among an integrin β4, an integrin β5, and an integrin β6.
(B4) The hybrid liposome exosome according to any one of (B1) to (B3), which is specifically delivered to pulmonary fibroblasts.
(B5) A method of forming a hybrid liposome exosome, the method including
an overexpression step in which an integrin α and an integrin β are overexpressed in cells;
a collection step in which an exosome is collected from the cells in which the integrin α and the integrin β are overexpressed; and
a hybrid liposome exosome forming step in which the collected exosome is fused with a liposome to form a hybrid liposome exosome.
(B6) The method of forming a hybrid liposome exosome according to (B5),
wherein the exosome collected in the collection step includes at least one exosome marker protein selected from among CD63, CD81 and CD9, and
wherein the exosome contains 0.01 ng or more and 100 ng or less of the integrin α and 0.01 ng or more and 100 ng or less of the integrin β per 1 ng of the total amount of the exosome marker protein present in the exosome.
(B7) The method of forming a hybrid liposome exosome according to (B5) or (B6),
wherein the cells are at least one selected from the group consisting of pluripotent stem cells, mesenchymal stem cells, ectoderm-derived cells, mesoderm-derived cells, and endoderm-derived cells.
(B8) A hybrid liposome exosome formed by the method of forming a hybrid liposome exosome according to any one of (B5) to (B7).
(B9) A hybrid liposome exosome-containing composition including the hybrid liposome exosome according to any one of (B1) to (B4) and a pharmaceutically acceptable carrier.
(B10) The hybrid liposome exosome-containing composition according to (B9), which is used to treat a lung disease, alleviate lung disease symptoms, and/or prevent a lung disease.

The present invention also includes the following inventions.
(C1) A method of forming a hybrid liposome exosome, the method including:
   an overexpression step in which an integrin β is overexpressed in cells;
   a collection step in which an exosome is collected from the cells in which the integrin β is overexpressed; and
   a hybrid liposome exosome forming step in which the collected exosome is fused with a liposome to form a hybrid liposome exosome.
(C2) The method of forming a hybrid liposome exosome according to (C1),
   wherein the exosome collected in the collection step includes an exosome marker protein,
   wherein the exosome marker protein is selected from among CD63, CD81 and CD9, and
   wherein the exosome contains 0.01 ng or more and 100 ng or less of the integrin β per 1 ng of the total amount of the exosome marker protein present in the exosome.
(C3) The method of forming a hybrid liposome exosome according to (C1) or (C2),
   wherein the cells are at least one selected from the group consisting of pluripotent stem cells, mesenchymal stem cells, ectoderm-derived cells, mesoderm-derived cells, and endoderm-derived cells.
(C4) A hybrid liposome exosome formed by the method of forming a hybrid liposome exosome according to any one of (C1) to (C3).
(C5) A hybrid liposome exosome including at least one exosome marker protein selected from among CD63, CD81 and CD9 and 0.01 ng or more and 100 ng or less of an integrin β per 1 ng of the total amount of the exosome marker protein present in the exosome in a lipid bilayer.
(C6) The hybrid liposome exosome according to (C5),
   wherein the exosome contains at least CD63 as the exosome marker protein, and
   wherein the hybrid liposome exosome contains 0.1 ng or more and 10 ng or less of the integrin β per 1 ng of the total amount of the exosome marker protein present in the exosome in the lipid bilayer.
(C7) The hybrid liposome exosome according to (C5) or (C6),
   wherein the integrin β is selected from among an integrin β4 and an integrin β5.
(C8) The hybrid liposome exosome according to any one of (C5) to (C7), targeting pulmonary fibroblasts.

The present invention also includes the following inventions.
(D1) A method of forming a hybrid liposome exosome, the method including:
   an overexpression step in which an integrin α is overexpressed in cells;
   a collection step in which an exosome is collected from the cells in which the integrin α is overexpressed; and
   a hybrid liposome exosome forming step in which the collected exosome is fused with a liposome to form a hybrid liposome exosome.
(D2) The method of forming a hybrid liposome exosome according to (D 1),
   wherein the exosome collected in the collection step includes an exosome marker protein,
   wherein the exosome marker protein is selected from among CD63, CD81 and CD9, and
   wherein the exosome contains 0.01 ng or more and 100 ng or less of the integrin α per 1 ng of the total amount of the exosome marker protein present in the exosome.
(D3) The method of forming a hybrid liposome exosome according to (D1) or (D2),
   wherein the cells are at least one selected from the group consisting of pluripotent stem cells, mesenchymal stem cells, ectoderm-derived cells, mesoderm-derived cells, and endoderm-derived cells.
(D4) A hybrid liposome exosome formed by the method of forming a hybrid liposome exosome according to any one of (D1) to (D3).
(D5) A hybrid liposome exosome including at least one exosome marker protein selected from among CD63, CD81 and CD9, and 0.01 ng or more and 100 ng or less of an integrin α per 1 ng of the total amount of the exosome marker protein present in the exosome in a lipid bilayer.
(D6) The hybrid liposome exosome according to (D5),
   wherein the exosome contains at least CD63 as the exosome marker protein, and
   wherein the hybrid liposome exosome contains 0.1 ng or more and 10 ng or less of the integrin α per 1 ng of the total amount of the exosome marker protein present in the exosome in the lipid bilayer.
(D7) The hybrid liposome exosome according to (D5) or (D6),
   wherein the integrin α is selected from among an integrin α3, an integrin α6, and an integrin α7.
(D8) The hybrid liposome exosome according to any one of (D5) to (D7), targeting vascular endothelial cells.

Since the integrin α and/or integrin β is expressed on the surface of the exosome, the exosome of the present invention can be specifically delivered to cells constituting specific tissues, and particularly, it is possible to provide the exosome suitable for delivery of drugs to the specific tissues in patients with specific tissue diseases.

### [Brief Description of Drawings]

[Fig. 1] Fig. 1 is a diagram illustrating an endosome formation and cell incorporation state.
[Fig. 2] Fig. 2 is a diagram showing a conceptual diagram of exosome flow cytometry using a phosphatidylserine-binding protein Tim4.
[Fig. 3] Fig. 3 is a diagram showing the results of a test of cell incorporation of integrin α gene-introduced HEK293F-derived exosomes into HUVEC cells in Experimental Example 1-4.
[Fig. 4] Fig. 4 is a diagram showing the results of a test of inhibition of cell incorporation of integrin α gene-introduced HEK293F-derived exosomes into HUVEC cells in Experimental Example 1-4 using antibodies.
[Fig. 5] Fig. 5 is a diagram showing the results of a test of cell incorporation of integrin α gene-introduced hybrid liposome exosomes into HUVEC cells in Experimental Example 1-5.
[Fig. 6] Fig. 6 is a diagram showing the results of a test of cell incorporation of integrin α gene- or integrin β gene-introduced HEK293F-derived exosomes into HPF cells in Experimental Example 2-3.
[Fig. 7] Fig. 7 is a diagram showing the results of a test of inhibition of cell incorporation of integrin α gene- or integrin β gene-introduced HEK293F-derived exosomes into HPF cells in Experimental Example 2-3 using antibodies.
[Fig. 8] Fig. 8 is a diagram showing the results of a test of cell incorporation of integrin α gene-introduced exosomes into HUVEC cells in Experimental Example 2-4.
[Fig. 9] Fig. 9 is a diagram showing the results of a test of cell incorporation of integrin α gene-introduced exosomes into HPF cells in Experimental Example 2-4.
[Fig. 10] Fig. 10 is a diagram showing the results of a test of cell incorporation of integrin α gene- or integrin β gene-introduced hybrid liposome exosomes into HPF cells in Experimental Example 2-5.
[Fig. 11] Fig. 11 is a diagram showing the results of a test of cell incorporation of integrin α gene- or integrin β gene-introduced hybrid liposome exosomes into HUVEC cells in Experimental Example 2-5.
[Fig. 12] Fig. 12 is a diagram showing the results of a test of cell incorporation of integrin α gene and/or integrin β gene-introduced HEK293F-derived exosomes into HPF cells in Experimental Example 3-3 and inhibition of cell incorporation using antibodies.
[Fig. 13] Fig. 13 is a diagram showing the results of a test of cell incorporation of integrin α gene and/or integrin β gene-introduced exosomes into HPF cells in Experimental Example 3-4.
[Fig. 14] Fig. 14 is a diagram showing the results of a test of cell incorporation of integrin α gene and/or integrin β gene-introduced exosomes into HUVEC cells in Experimental Example 3-4.
[Fig. 15] Fig. 15 is a diagram showing the results of a test of cell incorporation of integrin α gene- and/or integrin β gene- or integrin β gene-introduced hybrid liposome exosomes into HPF cells in Experimental Example 3-5.
[Fig. 16] Fig. 16 is a diagram showing the results of a test of cell incorporation of integrin α gene- and/or integrin β gene-introduced hybrid liposome exosomes into HUVEC cells in Experimental Example 3-5.
[Fig. 17] Fig. 17 is a diagram showing the results of proteome analysis of integrin-overexpressing exosomes in Experimental Example 4-1.
[Fig. 18] Fig. 18 is a diagram showing the results of a test of incorporation of PDIA4 knockdown HEK293 cell-derived exosomes into HPF cells in Experimental Example 4-2.

### [Description of Embodiments]

Hereinafter, forms for implementing the present invention will be described in detail with reference to the drawings. Here, embodiments of the present invention described below are only for examples, and the present invention is not limited to the embodiments described below.

### <Exosome>

The exosome of the present embodiment, which is an example of the present invention contains at least one exosome marker protein selected from among CD63, CD81, and CD9, and an integrin α and/or an integrin β in the lipid bilayer. According to preferable aspects of the present embodiment, the exosome of the present embodiment further contains a protein disulfide isomerase (PDI) in the lipid bilayer.

### [Exosome marker protein]

CD63, CD81, and CD9 are membrane proteins that are localized in the lipid bilayer of the exosome and are specifically found in the exosome. Hereinafter, characteristics of the proteins CD63, CD81, and CD9 will be simply described.

### (Characteristics of CD63)

CD63 is also known as lysosomal-associated membrane protein 3 (LAMP-3), and is a 30 kDa to 60 kDa lysosome membrane protein belonging to the tetraspanin family that has many important roles in immune-physiological functions. CD63 is known to mediate signal transmission related to regulation of cell development, activation, growth, and motility. Since CD63 is expressed on activated platelets, it has been pointed out that it may function as a platelet activation marker, and CD63 is a lysosome-associated membrane glycoprotein, and is known to translocate to the cell membrane after platelet activation. The amino acid sequence of human CD63 is listed as Accession No. P08962.2 in the sequence information database Genbank provided by the NCBI, and disclosed over the Internet. The content of the web page is incorporated as a part of this specification by reference.

### (Characteristics of CD81)

CD81 is a single-chain protein that belongs to the tetraspan family with four transmembrane domains. On the cell membrane, in CD81, both the N-terminal and the C-terminal are located in the cytoplasm, and two peptide loops are exposed outside the cell. CD81 does not contain sugar chains and has a molecular weight of 26 kDa. It has been pointed out that CD81 has a wide tissue distribution and is present in association with other tetraspan family members and the like. Immune B cells express relatively high levels of CD81 throughout their differentiation stages. The amino acid sequence of human CD81 is listed as Accession No. P60033.1 in the sequence information database Genbank provided by the NCBI and disclosed over the Internet. The content of the web page is incorporated as a part of this specification by reference.

### (Characteristics of CD9)

CD9 is a single-chain membrane protein having a molecular weight of 24 kDa and belongs to the tetraspan family. The CD9 antigen has four transmembrane domains and has a structure in which both the N-terminal and the C-terminal are present in the cell. It is found that CD9 is present in α granules of platelets, monocytes, pre-B cells, eosinophils, basophils, activated T cells and the like. It is thought that CD9 is associated with molecules such as very late activation (VLA) integrin molecules and HLA-DR, and involved in adhesion between cells, signal transmission, and cell motility. The amino acid sequence of human CD9 is listed as Accession No. P21926.4 in the sequence information database Genbank provided by the NCBI and disclosed over the Internet. The content of the web page is incorporated as a part of this specification by reference.

When the exosome of the present embodiment contains any one or two of three types of exosome marker proteins CD63, CD81 and CD9, predetermined effects of the present invention may be exhibited, but the exosome of the present embodiment preferably contains CD63 as an essential exosome marker protein, and more preferably contains all three types of exosome marker proteins CD63, CD81 and CD9.

### (Other exosome marker proteins)

Here, in addition to the exosome marker proteins localized in the lipid bilayer of the exosome, the exosome can also be identified using an exosome marker protein encapsulated in the exosome as a label. As such an exosome marker protein encapsulated in the exosome, for example, Alix may be exemplified.

ALG-2-interacting protein X (Alix) is a protein encoded by the PDCD6IP gene, also known as ALG-2-interacting protein 1 (AIP1) or Hp95. Alix is known to be involved in apoptosis (cell death) through a mechanism of action involving apoptosis linked gene 2 product (ALG-2). Here, ALG-2 is a 22 kDa protein having five repetitive EF-hand structures, and is known as a calcium-induced apoptosis regulator following endoplasmic reticulum (ER) stress. Alix is known to interact with ALG-2 through the C-terminal proline-rich region and to be involved in the formation of multi-vesicular bodies. The amino acid sequence of human Alix is listed as Accession No. Q8WUM4.1 in the sequence information database Genbank provided by the NCBI and disclosed over the Internet. The content of the web page is incorporated as a part of this specification by reference.

### (Integrin)

Integrins are cell membrane proteins known as cell adhesion molecules, and are known as proteins that have an important role in adhesion between cell-extracellular matrixes and adhesion between cells as extracellular matrix receptors. Protein molecules are heterodimers composed of two subunits of an α chain and a β chain, the α chain is also called an integrin α, the β chain is also called an integrin β, each chain has many subclasses, and various combinations of subclasses of the α chain and the β chain are possible. These proteins form a superfamily of integrins. It is known that, within cells, integrins bind to microfilaments of a cell skeleton via adapter proteins and transmit intracellular signals.

The exosome of the present embodiment contains an integrin α and/or an integrin β in the lipid bilayer. When the exosome contains the integrin α and/or the integrin β, it is possible to impart targeting to cells constituting specific tissues to the exosome of the present embodiment. When the exosome of the present embodiment contains the integrin α and the integrin β, the total amount of each integrin is preferably 0.01 ng or more and 100 ng or less per 1 ng of the total amount of the above exosome marker proteins (that is, one, two or all of CD63, CD81, and CD9). The upper limit value of the total amount of each of the integrin α and the integrin β is preferably 50 ng and 10 ng, and the lower limit value of the total amount of each of the integrin α and the integrin β is preferably 0.05 ng and 0.1 ng. When the total amount of each of the integrin α and the integrin β is within the above range, targeting of the exosome of the present embodiment to cells constituting specific tissues is enhanced, and non-specific delivery to other tissues is effectively inhibited. Particularly, when the total amount of each of the integrin α and the integrin β contained in the exosome of the present embodiment is 0.01 ng or more based on the above criteria, targeting can be more reliably expressed, and when the total amount is 100 ng or less, non-specific incorporation of exosomes can be more reliably avoided.

In the present embodiment, when both integrin α and integrin β are expressed in the exosome, the ratio of the expression levels of the integrin α and integrin β in the exosome is preferably 10:1 to 1:10 (integrin α:integrin β) and more preferably 2:1 to 1:2. Since the integrin α and the integrin β have a property of associating with each other, in the present embodiment, the expression levels of the integrin α and the integrin β is 1:1 in terms of the number of molecules, or the degree of deviation from this is particularly preferably within ±50%, for example, within ±40%, within ±30%, within ±20%, or within ±10%. When the expression ratio of the integrin α and the integrin β is adjusted, targeting of the exosome to cells constituting specific tissues becomes stronger.

Here, targeting of the exosome of the present embodiment generally differs depending on what kind of integrin is expressed in the exosome. For example, when only the integrin α is expressed in the exosome, targeting to vascular endothelial cells is enhanced, and when only the integrin β is expressed in the exosome, targeting to cells constituting lung tissues is enhanced. On the other hand, when both the integrin α and the integrin β are expressed in the exosome, targeting to cells constituting lung tissues is enhanced. Therefore, in the exosome of the present embodiment, depending on whether the integrin to be overexpressed is the integrin α, the integrin β or both, targeting of the exosome obtained as a result can be adjusted.

### (Method of measuring mass of protein)

Here, the mass of proteins contained in exosomes and hybrid liposome exosomes to be described below may be determined by general proteome analysis, and methods known to those skilled in the art can be used for such analysis. Here, general protocols for proteome analysis of membrane proteins and the like are described in Pharmaceutical Research, Vol. 25, No. 6, pp. 1469-1483, 2008, Journal of Proteome Research, Vol. 2, No. 1, pp. 43-50, 2003 and the like, the contents of which are incorporated into a part of this specification.

More specific examples of a proteome analysis method for the exosome of the present embodiment may include a proteome analysis method including the following steps. Here, in the following protocol, a Q Exactive mass spectrometer (commercially available from Thermo Fisher Scientific) is used as a mass spectrometer constituting LC-MS/MS, an UltiMate 3000 liquid chromatograph (commercially available from Thermo Fisher Scientific) is used as a liquid chromatograph, Proteome Discoverer 2.2 (commercially available from Thermo Fisher Scientific) is used as software for LC-MS/MS data integration and peptide detection intensity output, and Mascot ver. 2.5 (commercially available from Matrix Science) is used as software for comparing MS/MS data and amino acid sequence information and identifying peptides/proteins.

### Procedure 1. (acetone precipitation treatment)

A half volume (375 µL) of an exosome suspension is collected, and cold acetone in an amount 4 times the volume of the suspension is added to the collected solution and the mixture is then left overnight under conditions of -20°C. The supernatant produced by centrifugation (15,000×g, 4°C, for 15 minutes) is removed, and the precipitate is suspended in cold acetone. The suspension is mixed with inversion and centrifuged again (15,000×g, 4°C, for 5 minutes), and the supernatant is removed. Finally, the precipitate is air-dried (left at room temperature for 30 minutes). The dried precipitate is dissolved in a solubilization buffer solution (protein dissolution solution) (composition of solubilization buffer solution: 8 M urea, 50 mM Tris-HCl, pH 8.0).

### Procedure 2. (reductive alkylation)

The suspension is subjected to reduction with dithiothreitol, and then subjected to an alkylation treatment of cysteine residues with iodoacetamide. Procedure 3.

After the reductive alkylation treatment, 50 mM Tris-HCl (pH 8.0) is added, and the urea concentration in the solution is lowered to 2 M.

### Procedure 4. (hydrolysis reaction)

Trypsin is added and the mixture is incubated at 37°C for 16 hours. Procedure 5.

After the decomposition reaction, the peptide solution is subjected to a desalting treatment with C18 STAGE Tip (Rappsilber, J.et al., Anal. Chem. 2003, 75: 663-70).

### Procedure 6.

The sample after the desalting treatment is dried under a reduced pressure.

### Procedure 7. (LC-MS/MS of peptide mixture)

The desalted peptide mixture is subjected to LC-MS/MS (DDA). In LC, a 15 cm long particle-packed separation column is used. Here, first, one-tenth of the peptide mixture is measured, an optimal introduction amount is estimated and actual analysis is then performed.

### Procedure 8. (data processing)

The acquired MS/MS data is compared with a human amino acid sequence database.

Here, various subclasses of the integrin α and the integrin β are known. Particularly, the integrin α is preferably integrin α1 (for example, human integrin α1, GenBank Accession No. BC137121), integrin α2 (for example, human integrin α2, Uniprot Accession No. P17301), integrin α3 (for example, human integrin α3, GenBank Accession No. CH471109), integrin α4 (for example, human integrin α4, GenBank Accession No. CH471058), integrin α5 (for example, integrin α5, Uniprot Accession No. P08648), integrin α6 (for example, human integrin α6, GenBank Accession No. AK296496), integrin α7 (for example, human integrin α7, GenBank Accession No. AJ228836), integrin α8 (for example, human integrin α8, GenBank Accession No. AL590636), integrin α9 (for example, human integrin α9, GenBank Accession No. AP006240), integrin α10 (for example, human integrin α10, GenBank Accession No. AY358325), integrin α11 (for example, human integrin α11, GenBank Accession No. AC100825), integrin αL (for example, human integrin αL, GenBank Accession No. AC116348), integrin αX (for example, human integrin αX, GenBank Accession No. BC038237), integrin αM (for example, human integrin αM, GenBank Accession No. BC096346), integrin αD (for example, human integrin αD, GenBank Accession No. U40275), integrin αE (for example, human integrin αE, GenBank Accession No. BC117207), or integrin αv (for example, human integrin αv, Uniprot Accession No. P06756).

In addition, the integrin β is preferably integrin β1 (for example, human integrin β1, DB: Uniprot, Accession No. P05556), integrin β2 (for example, human integrin β2, DB: Uniprot, Accession No. P05107), integrin β3 (for example, human integrin β3, DB: Uniprot, Accession No. P05106), integrin β4 (for example, human integrin β4, DB: Uniprot, Accession No. P16144), integrin β5 (for example, human integrin β5, DB: Uniprot, Accession No. P18084), integrin β6 (for example, human integrin β6, DB: Uniprot, Accession No. P18564), integrin β7 (for example, human integrin β7, DB: Uniprot, Accession No. P26010), or integrin β8 (for example, human integrin β8, DB: Uniprot, Accession No. P26012). When the subclasses of these integrins β are expressed on the membrane surface of the exosome, it is easy to impart favorable targeting to cells constituting specific tissues.

Among the above subclasses of the integrins α, the integrin α3, the integrin α6, and the integrin α7 are more preferable, and the integrin α6 is particularly preferable. Here, the integrin α3, the integrin α6, and the integrin α7 are subclasses known to have high mutual homology. In addition, among the above subclasses of the integrin β, the integrin β1 and the integrin β4 are more preferable, and the integrin β4 is particularly preferable. Here, the integrin β1 and the integrin β4 are subclasses known to have high functional similarity to each other. Here, in the present embodiment, the combination of the subclass of the integrin α and the subclass of the integrin β is particularly preferably a combination of the integrin α6 and the integrin β4.

Here, while the exosome in which the integrin α and/or the integrin β itself is expressed is referred to in the present embodiment, the present invention is not necessarily limited to the exosome in which the integrin α and the integrin β themselves are expressed, and for example, exosomes in which mutant proteins having a sequence homology of at least 90%, 91%, 92%, 93%, 94%, 95%, 96%, 97%, 98%, 99%, 99.5%, or 99.9% with the subclasses of the integrin α and the integrin β are expressed are also included within the technical scope. Exosomes in which these mutant proteins are expressed can also have substantially the same effects as exosomes in which the subclasses of the integrin α and/or the integrin β itself are expressed. In the present embodiment, when simply referring to mutant proteins or mutant genes of the integrin α and/or the integrin β, they indicate mutant proteins having a sequence homology of at least 90% with the subclasses of the integrin α and/or the integrin β and mutant genes encoding such mutant proteins.

According to preferable aspects of the present embodiment, the exosome of the present embodiment contains a protein disulfide isomerase (PDI) in the lipid bilayer in addition to at least one exosome marker protein selected from among CD63, CD81 and CD9, the integrin α, and the integrin β. Although the present invention is not bound by any theory, when the exosome of the present embodiment contains the protein disulfide isomerase in the lipid bilayer in addition to the integrin α and the integrin β, the protein disulfide isomerase induces isomerization of disulfide bonds in the integrin α and the integrin β, more preferably, in the integrin β, and therefore conformational changes in the integrin α and the integrin β are induced and a change in binding of the integrin complex to the target is predicted. Therefore, in the present embodiment, when the exosome contains the protein disulfide isomerase in the lipid bilayer, it is inferred that the above targeting induced by the integrin α, the integrin β and the like is further enhanced and desired drugs are easily delivered to appropriate target cells.

Here, various subclasses of the protein disulfide isomerase (PDI) are known. Examples of subclasses of the protein disulfide isomerase (PDI) include protein disulfide isomerase A1 (PDIA1, DB: Uniprot, Accession No. P07237), protein disulfide isomerase A2 (PDIA2, DB: Uniprot, Accession No. H0Y4J5), protein disulfide isomerase A3 (PDIA3, DB: Uniprot, Accession No. P30101), protein disulfide isomerase A4 (PDIA4, DB: Uniprot, Accession No. P13667), protein disulfide isomerase A5 (PDIA5, DB: Uniprot, Accession No. Q14554), and protein disulfide isomerase A6 (PDIA6, DB: Uniprot, Accession No. Q15084).

Moreover, in addition to these, various subclasses described in Galligan and Petersen Human Genomics 2012, 6:6 Page 2 and the like are also known, the contents of which are incorporated into a part of this specification. More specifically, the protein disulfide isomerase (PDI) contained in the exosome of the present embodiment may be a class or subclass such as AGR2 (DB: Uniprot, Accession No. 095994), AGR3 (DB: Uniprot, Accession No. Q8TD06), CASQ1 (DB: Uniprot, Accession No. P31415), CASQ2 (DB: Uniprot, Accession No. 014958), DNAJC10 (DB: Uniprot, Accession No. Q8IXB1), ERP27 (DB: Uniprot, Accession No. Q96DN0), ERP29 (DB: Uniprot, Accession No. P30040), ERP44 (DB: Uniprot, Accession No. Q9BS26), PDILT (DB: Uniprot, Accession No. Q8N807), TMX1 (DB: Uniprot, Accession No. Q9H3N1), TMX2 (DB: Uniprot, Accession No. Q9Y320), TMX3 (DB: Uniprot, Accession No. Q96JJ7), TMX4 (DB: Uniprot, Accession No. Q9H1E5), TXNDC5 (DB: Uniprot, Accession No. Q8NBS9), and TXNDC12 (DB: Uniprot, Accession No. 095881).

Among the above examples, the protein disulfide isomerase is particularly preferably the protein disulfide isomerase A1, the protein disulfide isomerase A3, the protein disulfide isomerase A4, or the protein disulfide isomerase A5.

The content of the protein disulfide isomerase in the exosome of the present embodiment is preferably 0.01 ng or more and 100 ng or less per 1 ng of the total amount of at least one exosome marker protein selected from among CD63, CD81 and CD9 contained in the lipid bilayer. The lower limit value thereof is more preferably 0.02 ng, 0.05 ng, 0.1 ng, 0.5 ng, 1.0 ng, 5.0 ng, or 10 ng, and the upper limit value thereof is more preferably 20 ng, 30 ng, 40 ng, 50 ng, 60 ng, 70 ng, 80 ng, or 90 ng. When the exosome contains the protein disulfide isomerase in the above content, targeting of the exosome to target cells becomes better.

In addition, the content of the protein disulfide isomerase in the exosome of the present embodiment is preferably 0.01 ng or more and 100 ng or less per 1 ng of the total amount of phospholipids contained in the lipid bilayer. The lower limit value thereof is more preferably 0.02 ng, 0.05 ng, 0.1 ng, 0.5 ng, 1.0 ng, 5.0 ng, or 10 ng, and the upper limit value thereof is preferably 20 ng, 30 ng, 40 ng, 50 ng, 60 ng, 70 ng, 80 ng, or 90 ng. When the exosome contains the protein disulfide isomerase in the above content, targeting of the exosome to target cells becomes better.

In addition, the molar ratio of the protein disulfide isomerase (PDI), the integrin α and the integrin β in the exosome of the present embodiment is preferably 1:0.1 to 1:10.

Examples of the preferable protein disulfide isomerase (PDI), the integrin α and the integrin β are shown in the table below, along with their amino acid sequences.

**[Table 1-1]**

| |
|---|
| human PDI A4/Uniprot Entry: P13667/used in Experimental Examples 4-1&4-2/SEQ ID No. 6 |
| MRPRKAFLLLLLLGLVQLLAVAGAEGPDEDSSNRENAIEDEEEEEEEDDDEEEDDLEVKEENGVLVLNDANFDN FVADKDTVLLEFYAPWCGHCKQFAPEYEKIANILKDKDPPIPVAKIDATSASVLASRFDVSGYPTIKILKKGQAVD YEGSRTQEEIVAKVREVSQPDWTPPPEVTLVLTKENFDEVVNDADIILVEFYAPWCGHCKKLAPEYEKAAKELSK RSPPIPLAKVDATAETDLAKRFDVSGYPTLKIFRKGRPYDYNGPREKYGIVDYMIEQSGPPSKEILTLKQVQEFLK DGDDVIIIGVFKGESDPAYQQYQDAANNLREDYKFHHTFSTEIAKFLKVSQGQLVVMQPEKFQSKYEPRSHMMD VQGSTQDSAIKDFVLKYALPLVGHRKVSNDAKRYTRRPLVVVYYSVDFSFDYRAATQFWRSKVLEVAKDFPEY TFAIADEEDYAGEVKDLGLSESGEDVNAAILDESGKKFAMEPEEFDSDTLREFVTAFKKGKLKPVIKSQPVPKNN KGPVKVVVGKTFDSIVMDPKKDVLIEFYAPWCGHCKQLEPVYNSLAKKYKGQKGLVIAKMDATANDVPSDRYK VEGFPTIYFAPSGDKKNPVKFEGGDRDLEHLSKFIEEHATKLSRTKEEL |
| human PDI A1/Uniprot Entry: P07237/SEQ ID No. 7 |
| |
| human PDI A3/Uniprot Entry: P30101/SEQ ID No. 8 |
| MRLRRLALFPGVALLLAAARLAAASDVLELTDDNFESRISDTGSAGLMLVEFFAPWCGHCKRLAPEYEAAATRL KGIVPLAKVDCTANTNTCNKYGVSGYPTLKIFRDGEEAGAYDGPRTADGIVSHLKKQAGPASVPLRTEEEFKKFI SDKDASIVGFFDDSFSEAHSEFLKAASNLRDNYRFAHTNVESLVNEYDDNGEGIILFRPSHLTNKFEDKTVAYTE QKMTSGKIKKFIQENIFGICPHMTEDNKDLIQGKDLLIAYYDVDYEKNAKGSNYWRNRVMMVAKKFLDAGHKLNF AVASRKTFSHELSDFGLESTAGEIPVVAIRTAKGEKFVMQEEFSRDGKALERFLQDYFDGNLKRYLKSEPIPESN DGPVKVVVAENFDEIVNNENKDVLIEFYAPWCGHCKNLEPKYKELGEKLSKDPNIVIAKMDATANDVPSPYEVR GFPTIYFSPANKKLNPKKYEGGRELSDFISYLQREATNPPVIQEEKPKKKKKAQEDL |
| human PDI A6/Uniprot Entry: Q15084/SEQ ID No. 9 |
| MALLVLGLVSCTFFLAVNGLYSSSDDVIELTPSNFNREVIQSDSLWLVEFYAPWCGHCQRLTPEWKKAATALKD VVKVGAVDADKHHSLGGQYGVQGFPTIKIFGSNKNRPEDYQGGRTGEAIVDAALSALRQLVKDRLGGRSGGYS SGKQGRSDSSSKKDVIELTDDSFDKNVLDSEDVWMVEFYAPWCGHCKNLEPEWAAAASEVKEQTKGKVKLAA VDATVNQVLASRYGIRGFPTIKIFQKGESPVDYDGGRTRSDIVSRALDLFSDNAPPPELLEIINEDIAKRTCEEHQL CVVAVLPHILDTGAAGRNSYLEVLLKLADKYKKKMWGWLWTEAGAQSELETALGIGGFGYPAMAAINARKMKF ALLKGSFSEQGINEFLRELSFGRGSTAPVGGGAFPTIVEREPWDGRDGELPVEDDIDLSDVELDDLGKDEL |
| human integrin *α*4/Uniprot Entry: P13612/SEQ ID No. 10 |
| |

**[Table 1-2]**

| |
|---|
| human integrin *α*v/Uniprot Entry: P06756/used in Experimental Examples 1-2&2-1&2-3&3-1&4-1/SEQ ID No. 11 |
| MAFPPRRRLRLGPRGLPLLLSGLLLPLCRAFNLDVDSPAEYSGPEGSYFGFAVDFFVPSASSRMFLLVGAPKA NTTQPGIVEGGQVLKCDWSSTRRCQPIEFDATGNRDYAKDDPLEFKSHQWFGASVRSKQDKILACAPLYHWRT EMKQEREPVGTCFLQDGTKTVEYAPCRSQDIDADGQGFCQGGFSIDFTKADRVLLGGPGSFYWQGQLISDQV AEIVSKYDPNVYSIKYNNQLATRTAQAIFDDSYLGYSVAVGDFNGDGIDDFVSGVPRAARTLGMVYIYDGKNMS SLYNFTGEQMAAYFGFSVAATDINGDDYADVFIGAPLFMDRGSDGKLQEVGQVSVSLQRASGDFQTTKLNGFE VFARFGSAIAPLGDLDQDGFNDIAIAAPYGGEDKKGIVYIFNGRSTGLNAVPSQILEGQWAARSMPPSFGYSMK GATDIDKNGYPDLIVGAFGVDRAILYRARPVITVNAGLEVYPSILNQDNKTCSLPGTALKVSCFNVRFCLKADGK GVLPRKLNFQVELLLDKLKQKGAIRRALFLYSRSPSHSKNMTISRGGLMQCEELIAYLRDESEFRDKLTPITIFME YRLDYRTAADTTGLQPILNQFTPANISRQAHILLDCGEDNVCKPKLEVSVDSDQKKIYIGDDNPLTLIVKAQNQG EGAYEAELIVSIPLQADFIGVVRNNEALARLSCAFKTENQTRQVVCDLGNPMKAGTQLLAGLRFSVHQQSEMD TSVKFDLQIQSSNLFDKVSPVVSHKVDLAVLAAVEIRGVSSPDHVFLPIPNWEHKENPETEEDVGPVVQHIYELR NNGPSSFSKAMLHLQWPYKYNNNTLLYILHYDIDGPMNCTSDMEINPLRIKISSLQTTEKNDTVAGQGERDHLIT KRDLALSEGDIHTLGCGVAQCLKIVCQVGRLDRGKSAILYVKSLLWTETFMNKENQNHSYSLKSSASFNVIEFPY KNLPIEDITNSTLVTTNVTWGIQPAPMPVPVWVIILAVLAGLLLLAVLVFVMYRMGFFKRVRPPQEEQEREQLQP HENGEGNSET |
| human integrin *α*6/Uniprot Entry: P23229/used in Experimental Examples 1-2&2-1&2-3&3-1&4-1/SEQ ID No. 12 |
| MAAAGQLCLLYLSAGLLSRLGAAFNLDTREDNVIRKYGDPGSLFGFSLAMHWQLQPEDKRLLLVGAPRAEALP LQRANRTGGLYSCDITARGPCTRIEFDNDADPTSESKEDQWMGVTVQSQGPGGKVVTCAHRYEKRQHVNTKQ ESRDIFGRCYVLSQNLRIEDDMDGGDWSFCDGRLRGHEKFGSCQQGVAATFTKDFHYIVFGAPGTYNWKGIVR VEQKNNTFFDMNIFEDGPYEVGGETEHDESLVPVPANSYLGLLFLTSVSYTDPDQFVYKTRPPREQPDTFPDV MMNSYLGFSLDSGKGIVSKDEITFVSGAPRANHSGAVVLLKRDMKSAHLLPEHIFDGEGLASSFGYDVAVVDLN KDGWQDIVIGAPQYFDRDGEVGGAVYVYMNQQGRWNNVKPIRLNGTKDSMFGIAVKNIGDINQDGYPDIAVGA PYDDLGKVFIYHGSANGINTKPTQVLKGISPYFGYSIAGNMDLDRNSYPDVAVGSLSDSVTIFRSRPVINIQKTITV TPNRIDLRQKTACGAPSGICLQVKSCFEYTANPAGYNPSISIVGTLEAEKERRKSGLSSRVQFRNQGSEPKYTQ ELTLKRQKQKVCMEETLWLQDNIRDKLRPIPITASVEIQEPSSRRRVNSLPEVLPILNSDEPKTAHIDVHFLKEGC GDDNVCNSNLKLEYKFCTREGNQDKFSYLPIQKGVPELVLKDQKDIALEITVTNSPSNPRNPTKDGDDAHEAKLI ATFPDTLTYSAYRELRAFPEKQLSCVANQNGSQADCELGNPFKRNSNVTFYLVLSTTEVTFDTPDLDINLKLET TSNQDNLAPITAKAKVVIELLLSVSGVAKPSQVYFGGTVVGEQAMKSEDEVGSLIEYEFRVINLGKPLTNLGTAT LNIQWPKEISNGKWLLYLVKVESKGLEKVTCEPQKEINSLNLTESHNSRKKREITEKQIDDNRKFSLFAERKYQTL NCSVNVNCVNIRCPLRGLDSKASLILRSRLWNSTFLEEYSKLNYLDILMRAFIDVTAAAENIRLPNAGTQVRVTVF PSKTVAQYSGVPWWIILVAILAGILMLALLVFILWKCGFFKRSRYDDSVPRYHAVRIRKEEREIKDEKYIDNLEKKQ WITKWNENESYS |

**[Table 1-3]**

| |
|---|
| human integrin *β*4/Uniprot Entry: P16144/SEQ ID No. 13 |
| MAGPRPSPWARLLLAALISVSLSGTLANRCKKAPVKSCTECVRVDKDCAYCTDEMFRDRRCNTQAELLAAGC QRESIVVMESSFQITEETQIDTTLRRSQMSPQGLRVRLRPGEERHFELEVFEPLESPVDLYILMDFSNSMSDDLD NLKKMGQNLARVLSQLTSDYTIGFGKFVDKVSVPQTDMRPEKLKEPWPNSDPPFSFKNVISLTEDVDEFRNKL QGERISGNLDAPEGGFDAILQTAVCTRDIGWRPDSTHLLVFSTESAFHYEADGANVLAGIMSRNDERCHLDTTG TYTQYRTQDYPSVPTLVRLLAKHNIIPIFAVTNYSYSYYEKLHTYFPVSSLGVLQEDSSNIVELLEEAFNRIRSNLD IRALDSPRGLRTEVTSKMFQKTRTGSFHIRRGEVGIYQVQLRALEHVDGTHVCQLPEDQKGNIHLKPSFSDGLK MDAGIICDVCTCELQKEVRSARCSFNGDFVCGQCVCSEGWSGQTCNCSTGSLSDIQPCLREGEDKPCSGRGE CQCGHCVCYGEGRYEGQFCEYDNFQCPRTSGFLCNDRGRCSMGQCVCEPGWTGPSCDCPLSNATCIDSNG GICNGRGHCECGRCHCHQQSLYTDTICEINYSAIHPGLCEDLRSCVQCQAWGTGEKKGRTCEECNFKVKMVDE LKRAEEVVVRCSFRDEDDDCTYSYTMEGDGAPGPNSTVLVHKKKDCPPGSFWWLIPLLLLLLPLLALLLLLCWK YCACCKACLALLPCCNRGHMVGFKEDHYMLRENLMASDHLDTPMLRSGNLKGRDVVRWKVTNNMQRPGFAT HAASINPTELVPYGLSLRLARLCTENLLKPDTRECAQLRQEVEENLNEVYRQISGVHKLQQTKFRQQPNAGKKQ DHTIVDTVLMAPRSAKPALLKLTEKQVEQRAFHDLKVAPGYYTLTADQDARGMVEFQEGVELVDVRVPLFIRP EDDDEKQLLVEAIDVPAGTATLGRRLVNITIIKEQARDVVSFEQPEFSVSRGDQVARIPVIRRVLDGGKSQVSYR TQDGTAQGNRDYIPVEGELLFQPGEAWKELQVKLLELQEVDSLLRGRQVRRFHVQLSNPKFGAHLGQPHSTTII IRDPDELDRSFTSQMLSSQPPPHGDLGAPQNPNAKAAGSRKIHFNWLPPSGKPMGYRVKYWIQGDSESEAHLL DSKVPSVELTNLYPYCDYEMKVCAYGAQGEGPYSSLVSCRTHQEVPSEPGRLAFNVVSSTVTQLSWAEPAET NGEITAYEVCYGLVNDDNRPIGPMKKVLVDNPKNRMLLIENLRESQPYRYTVKARNGAGWGPEREAIINLATQPK RPMSIPIIPDIPIVDAQSGEDYDSFLMYSDDVLRSPSGSQRPSVSDDTGCGWKFEPLLGEELDLRRVTWRLPPEL IPRLSASSGRSSDAEAPHGPPDDGGAGGKGGSLPRSATPGPPGEHLVNGRMDFAFPGSTNSLHRMTTTSAAA YGTHLSPHVPHRVLSTSSTLTRDYNSLTRSEHSHSTTLPRDYSTLTSVSSHDSRLTAGVPDTPTRLVFSALGP TSLRVSWQEPRCERPLQGYSVEYQLLNGGELHRLNIPNPAQTSVVVEDLLPNHSYVFRVRAQSQEGWGRERE GVITIESQVHPQSPLCPLPGSAFTLSTPSAPGPLVFTALSPDSLQLSWERPRRPNGDIVGYLVTCEMAQGGGP ATAFRVDGDSPESRLTVPGLSENVPYKFKVQARTTEGFGPEREGIITIESQDGGPFPQLGSRAGLFQHPLQSEY SSITTTHTSATEPFLVDGLTLGAQHLEAGGSLTRHVTQEFVSRTLTTSGTLSTHMDQQFFQT |
| human integrin *β*5/Uniprot Entry: P18084/used in Experimental Examples 2-1&2-3&3-1&4-1/SEQ ID No. 14 |
| MPRAPAPLYACLLGLCALLPRLAGLNICTSGSATSCEECLLIHPKCAWCSKEDFGSPRSITSRCDLRANLVKNG CGGEIESPASSFHVLRSLPLSSKGSGSAGWDVIQMTPQEIAVNLRPGDKTTFQLQVRQVEDYPVDLYYLMDLSL SMKDDLDNIRSLGTKLAEEMRKLTSNFRLGFGSFVDKDISPFSYTAPRYQTNPCIGYKLFPNCVPSFGFRHLLPL TDRVDSFNEEVRKQRVSRNRDAPEGGFDAVLQAAVCKEKIGWRKDALHLLVFTTDDVPHIALDGKLGGLVQPH DGQCHLNEANEYTASNQMDYPSLALLGEKLAENNINLIFAVTKNHYMLYKNFTALIPGTTVEILDGDSKNIIQLIIN AYNSIRSKVELSVWDQPEDLNLFFTATCQDGVSYPGQRKCEGLKIGDTASFEVSLEARSCPSRHTEHVFALRPV GFRDSLEVGVTYNCTCGCSVGLEPNSARCNGSGTYVCGLCECSPGYLGTRCECQDGENQSVYQNLCREAEG KPLCSGRGDCSCNQCSCFESEFGKIYGPFCECDNFSCARNKGVLCSGHGECHCGECKCHAGYIGDNCNCSTDI STCRGRDGQICSERGHCLCGQCQCTEPGAFGEMCEKCPTCPDACSTKRDCVECLLLHSGKPDNQTCHSLCR DEVITWVDTIVKDDQEAVLCFYKTAKDCVMMFTYVELPSGKSNLTVLREPECGNTPNAMTILLAVVGSILLVGLA LLAIWKLLVTIHDRREFAKFQSERSRARYEMASNPLYRKPISTHTVDFTFNKFNKSYNGTVD |
| human integrin *β*6/Uniprot Entry: P18564/used in Experimental Examples 2-1&2-3&3-1&4-1/SEQ ID No. 15 |
| |

The proteins used in the present embodiment may be mutant proteins including an amino acid sequence having a sequence homology of at least 90%, 91%, 92%, 93%, 94%, 95%, 96%, 97%, 98%, 99%, 99.5%, or 99.9% with an amino acid sequence of original proteins and having the same functions as the original proteins (for example, functions as a PDI, integrin α or integrin β). In this specification, "sequence homology" may be determined by a sequence comparison program known in the art, and for example, may be determined using default parameters in the BLAST program.

### <Method of forming exosome and hybrid liposome exosome>

The present invention relates to a method of forming an exosome and a hybrid liposome exosome. That is, in the present embodiment, the above exosome may be used without change, a hybrid liposome exosome may be formed from the above exosome, and the hybrid liposome exosome may be used. The method of forming an exosome of the present embodiment is a method of forming the above exosome of the present embodiment, the method including an overexpression step in which an integrin α and/or an integrin β, and/or a mutant protein having a sequence homology of at least 90% therewith are overexpressed in cells, and a collection step in which an exosome is collected from the cells in which the integrin α and/or the integrin β, and/or the mutant protein having a sequence homology of at least 90% therewith are overexpressed. In addition, the method of forming a hybrid liposome exosome of the present embodiment is a method of forming the hybrid liposome exosome of the present embodiment, including a hybrid liposome exosome forming step in which the collected exosome is fused with a liposome to form a hybrid liposome exosome in the method of forming an exosome of the present embodiment.

### [Overexpression step]

In order to impart targeting to the cells constituting specific tissues to the exosome of the present embodiment, an overexpression step in which an integrin α and/or an integrin β, and/or a mutant protein having a sequence homology of at least 90% therewith are overexpressed in cells from which an exosome to be described below is derived is performed by a method to be described below. Detailed and specific methods for overexpressing an integrin α and/or an integrin β, and/or a mutant protein having a sequence homology of at least 90% therewith in cells are disclosed in, for example, Brian Dalby et al., "Advanced transfection with Lipofectamine 2000 reagent: primary neurons, siRNA, and high-throughput applications," Methods, Vol. 33, Issue 2, p. 95-103 (2004) and the like, the contents of which are incorporated as a part of this specification by reference. More specifically, a vector incorporating one or two or more gene fragments encoding an integrin α and/or an integrin β, and/or a mutant protein having a sequence homology of at least 90% therewith is transfected into cells to be described below to induce expression of the integrin α gene and/or the integrin β gene, and/or their mutant genes under expression induction conditions determined by the type of promoters of the vector. Here, transfection of the integrin α gene, the integrin β gene and the like into cells does not necessarily have to be performed using one type of vector, and the integrin α and the integrin β may be expressed by separately transfecting cells with a vector into which the integrin α is introduced and a vector into which the integrin β is introduced. However, in order to adjust the ratio of the expression levels of the integrin α and the integrin β, it is preferable to introduce two types of genes including the integrin α gene and the integrin β gene into a single vector and express the genes under the control of the same promoter or a promoter with an equivalent expression level.

### (Type of expression vector)

In the method of forming an exosome of the present embodiment, as the vector used in the overexpression step, expression vectors generally known as expression vectors for gene expression in mammals can be used. More specifically, plasmids derived from E. coli such as pBR322, pBR325, pUC12, and pUC13; plasmids derived from Bacillus subtilis such as pUB110, pTPS, and pC194; plasmids derived from yeasts such as pSH19 and pSH15; bacteriophages such as λ phase; viruses such as adenovirus, adeno-associated virus, lentivirus, vaccinia virus, and baculovirus; and vectors modified from these can be used. The conditions for transfecting the following cells with these expression vectors may be standard transfection conditions commonly used by those skilled in the art.

### (Structure of expression vector)

The expression vector may contain nucleotide sequences encoding each of the integrin α, the integrin β, and their mutant proteins, and preferably a promoter and an enhancer operably linked to a double-stranded DNA sequence.

Here, "operably linked" refers to a functional link between gene expression control sequences (for example, transcription factor binding sites such as promoters and enhancers) and genes desired to be expressed (in the present embodiment, polynucleotides encoding each of the integrin α, the integrin β, and their mutant proteins). Here, "expression control sequence" refers to a sequence that directs transcription of genes desired to be expressed (in the present embodiment, polynucleotides encoding each of the integrin α, the integrin β, and their mutant proteins).

Here, when polynucleotide sequences encoding each of the integrin α and the integrin β are incorporated into the above vectors, the full length of the integrin α gene and the integrin β gene may be incorporated, or a part of the sequence (for example, a sequence of 90% or more of an encoding region encoding the integrin α and the integrin β, and more preferably, a sequence of 91% or more, 92% or more, 93% or more, 94% or more, 95% or more, 96% or more, 97% or more, 98% or more, or 99% or more thereof) may be incorporated. However, when a part of the sequence of the integrin α and the integrin β is incorporated into the vector, it is preferable to construct the polynucleotide sequence so that the nucleotide sequence encoding the 3 amino acid residues to 65 amino acid residues at the N-terminal is necessarily incorporated into the vector in order to prevent signal peptides such as membrane translocation signals from being lost.

### (Promoter)

The promoter to be incorporated into the expression vector is not particularly limited, and can be appropriately determined depending on, for example, the type of cells, and expression conditions. Specific examples of promoters include, for example, viral promoters, expression-inducible promoters, tissue-specific promoters, and promoters fused with other promoter sequences or enhancer sequences. The promoter is preferably linked to the upstream (5' end side) of the polynucleotide encoding the integrin α, the integrin β, and their mutant proteins.

Here, "viral promoter" is a virus-derived promoter. Examples of originating viruses include cytomegalovirus, Moloney leukemia virus, JC virus, breast cancer virus, simian virus, and retrovirus.

In addition, the "expression-inducible promoter" is a promoter that allows a gene (in the present embodiment, the integrin α gene and the integrin β gene) desired to be expressed when a specific stimulus such as a chemical agent and physical stress is applied to be expressed, and does not exhibit expression activity in the absence of a stimulus. Examples of expression-inducible promoters include TetO (tetracycline operator) promoters, metallothionein promoters, IPTG/lacI promoters, ecdysone promoters, and "lox stop lox" for irreversibly deleting an inhibitory sequence for translation or transcription, but the present invention is not limited thereto.

"Tissue-specific promoter" refers to a promoter having expression activity only in specific tissues.

Promoters fused with other promoter sequences or enhancer sequences include, for example, the SRα promoter composed of an early gene promoter of simian virus 40 (SV40) and a partial sequence of the long terminal repeat of human T-cell leukemia virus 1, and the CAG promoter composed of a cytomegalovirus immediate-early (IE) gene enhancer and a chicken β-actin promoter. Particularly, the CAG promoter allows a polyA signal site of a rabbit β-globin gene to be added at the 3' end of a gene desired to be expressed (in the present embodiment, the α integrin gene) and allows the gene desired to be expressed (in the present embodiment, the α integrin gene) to be overexpressed in almost all types of cells.

Here, in the above expression vector, furthermore, for example, a polyadenylation signal necessary for polyadenylation of the 3' end of mRNA may be operably linked to the downstream (3' end side) of the integrin α gene, the integrin β gene, and their mutant genes. Polyadenylation signals include polyadenylation signals contained in genes derived from the above viruses and derived from various humans or non-human animals, and for example, polyadenylation signals of late genes or early genes of SV40, rabbit β-globin genes, bovine growth hormone genes, and human A3 adenosine receptor genes may be exemplified.

### (Obtaining exosome)

In the present embodiment, exosomes are obtained from any cell selected from the group consisting of pluripotent stem cells, mesenchymal stem cells, ectoderm-derived cells, mesoderm-derived cells, and endoderm-derived cells. Therefore, in the present embodiment, since exosomes derived from cells with various properties can be used, various cell characteristics can be imparted to the exosomes or hybrid liposome exosomes depending on characteristics of the exosomes.

As pluripotent stem cells, mesenchymal stem cells, ectoderm-derived cells, mesoderm-derived cells, and endoderm-derived cells that can be used in the method of forming an exosome or a hybrid liposome exosome of the present embodiment, for example, the following cells may be exemplified.

### (Pluripotent stem cells)

Pluripotent stem cells are cells that potentially have an ability to differentiate into various tissues of a living body, and specifically, cells that can differentiate into any of endoderm, mesoderm, and ectoderm. Examples of such cells include embryonic stem cells (ES cells) and induced pluripotent stem cells (iPS cells).

### (Mesenchymal stem cells)

Mesenchymal stem cells are stem cells that have an ability to differentiate into tissues mainly derived from mesoderm, tissues derived from some ectoderm and endodermal tissue. As markers for mesenchymal stem cells, adhesive molecules CD106, CD166, and CD29, and CD105, CD73, CD44, CD90, and CD71 are known, and as negative markers, adhesive molecules CD31, CD18, and CD56, hematopoietic markers CD45, CD34, CD14, and CD11, and co-stimulation molecules CD80, CD86, and CD40 are known.

### (Ectoderm-derived cells)

Examples of ectoderm-derived cells include keratinizing epithelial cells (epidermal keratinocytes, epidermal basal cells, nail keratin-producing cells, nail bed basal cells, medullary hair stem cells, cortical hair stem cells, cuticular hair stem cells, cuticular root sheath cells, Huxley layer root sheath cells, Henle layer root sheath cells, outer root sheath cells, and hair follicle cells), stratified epithelial cells (single-layer squamous epithelial cells of the cornea, tongue, oral cavity, esophagus, anal canal, urethra, and vagina; basal cells of the cornea, tongue, oral cavity, esophagus, anal canal, urethra, and vagina; and bladder epidermal cells), inner hair cells of the organ of Corti, outer hair cells of the organ of Corti, basal cells of the olfactory epithelium, cold sensory neurons, thermosensory sensory neurons, Merkel cells of the epidermis, olfactory receptor neurons, pain sensory neurons, retinal photoreceptor cells (rod cells, blue cone cells, green cone cells, red cone cells), deep sensory neurons, tactile sensory neurons, type I glomus cells, type II glomus cells, type I hair cells of the vestibular system, type II hair cells of the vestibular system, type I taste bud cells, autonomic nerve cells (cholinergic neurons, adrenergic neurons, and peptidergic neurons), inner pillar cells of the organ of Corti, outer pillar cells of the organ of Corti, inner phalangeal cells of the organ of Corti, outer phalangeal cells of the organ of Corti, border cells of the organ of Corti, Hensen's cells of the organ of Corti, vestibular organ supporting cells, taste bud supporting cells, olfactory epithelial supporting cells, Schwann cells, satellite cells, enteric glial cells, central nervous system neurons and glial cells (astrocytes, neurons, oligodendrocytes, and spindle neurons), and lens cells (anterior lens epidermal cells, and crystalline lens fiber cells).

### (Mesoderm-derived cells)

Examples of mesoderm-derived cells include hepatocytes, adipocytes (white adipocytes, and brown adipocytes), Ito cells, pararenal cells, glomerular epithelial cells, proximal tubular brush border cells, thin segment cells of the loop of Henle, distal tubule cells, collecting tubule cells, type I pneumocyte epithelial cells, centroacinar cells, smooth muscle duct cells (principal cells, and intercalated cells), duct cells, intestinal brush border cells, exocrine striatal duct cells, gallbladder epithelial cells, testicular efferent duct non-ciliated cells, epididymal principal cells, epididymal basal cells, ameloblasts, meniscal epithelial cells, interdental epithelial cells of the organ of Corti, loose connective tissue fibroblasts, corneal fibroblasts, tendon fibroblasts, bone marrow reticular connective tissue fibroblasts, other non-epithelial fibroblasts, pericytes, nucleus pulposus cells of the intervertebral disc, cementoblasts, odontoblasts, hyaline cartilage chondrocytes, fibrocartilage chondrocytes, elastic cartilage chondrocytes, osteoblasts, osteoprogenitor cells, hyalocytes of the vitreous body, stellate cells of the ear, pancreatic stellate cells, contractile cells, skeletal muscle cells (slow muscle cells, fast muscle cells, intermediate muscle cells, nuclear bag fibers of muscle spindles, and nuclear chain fibers of muscle spindles), satellite cells, cardiomyocytes (cardiomyocytes, segmented cardiomyocytes, and Purkinje fiber cells), smooth muscle cells, myoepithelial cells of the iris, myoepithelial cells of the exocrine glands, megakaryocytes, monocytes, connective tissue macrophages, epithelial Langerhans cells, osteoclasts, dendritic cells, microglial cells, neutrophils, eosinophils, basophils, hybridoma cells, mast cells, Th2 cells, regulatory T cells, cytotoxic T cells, natural killer T cells, B cells, natural killer cells, reticulocytes, stem cells and progenitor cells of the blood and immune system, erythroblasts, myelocytes, oocytes, sperm cells, spermatocytes, spermatogenous cells, nurse cells, follicular ovarian cells, Sertoli cells, thymic epithelial cells, interstitial kidney cells, and any cultured cells derived therefrom.

### (Endoderm-derived cells)

Examples of endoderm-derived cells include salivary gland mucus cells, salivary gland (n1) cells, von Ebner gland cells of the taste buds, mammary gland cells, lacrimal gland cells, auditory canal gland cells, eccrine glands, glandular dark cells, eccrine gland clear cells, apocrine gland cells, eyelash gland cells, sebaceous gland cells, Bowman's gland cells of the epithelial cells of the nose, Brunner's gland cells of the duodenum, seminal vesicle cells, prostate cells, bulbourethral gland cells, Bartholin gland cells, urethral gland cells, endometrial cells, goblet cells, gastric mucosal cells, principal cells of the stomach, parietal cells, pancreatic acinar cells, paneth cells of the small intestine, type II pneumocytes of the lungs, clara cells of the lungs, anterior pituitary cells (growth hormone-secreting cells, prolactin-secreting cells, pituitary-tropic thyroid cells, gonadotropic cells, and adrenocorticotrophic hormone-secreting cells), melanocyte-stimulating hormone-producing cells, large neurosecretory neurons, thyroid cells (follicular cells, and parafollicular cells), parathyroid cells (principal cells, and acidophilic cells), adrenal cells (chromaffin cells, etc.), Leydig cells, internal capsule cells, luteal cells (granulosa lutein cells, and theca lutein cells), juxtaglomerular cells, macula densa cells, circumpolar cells, mesangial cells, and any cultured cells derived therefrom.

### [Collection step]

The method of forming an exosome of the present embodiment includes a collection step in which an exosome is collected from cells overexpressing integrin α, integrin β, and/or their mutant genes. In the collection step, in order to obtain exosomes from the above cells, these cells are cultured for 1 day to 30 days in a general medium commonly used for cell culture, the culture solution is collected, as necessary, and exosomes with a particle size of 20 nm to 150 nm (in diameter) can be obtained by separation and concentration by conventionally commonly used column chromatography such as gel filtration chromatography, affinity column chromatography using antibodies against antigens selected from among the above CD63, CD81, and CD9, size exclusion chromatography, and phosphatidylserine affinity chromatography or flow cytometry. Here, it is clear that the culture supernatant containing an exosome does not contain any solid component other than the exosome, and if the exosome concentration operation is not required depending on characteristics of an operation to be performed, the above separation and concentration operation may not be performed.

Here, the medium that can be used in cell culture for obtaining exosomes is not particularly limited and any general cell culture medium can be used. However, in order to increase the exosome production ability, for example, it is preferable to use a completely synthetic medium that does not contain an exosome, and when a natural medium is used or even when a naturally derived component such as serum is added to a synthetic medium, it is preferable that an exosome be removed from the natural medium or naturally derived raw materials.

### [Hybrid liposome exosome forming step]

As described above, the exosome of the present embodiment may be fused with a liposome to form a hybrid liposome exosome. The method of forming a hybrid liposome exosome of the present embodiment includes a hybrid liposome exosome forming step in which the collected exosome is fused with a liposome to form a hybrid liposome exosome.

### (Formation of liposome)

When a liposome is formed, for example, commercially available liposome preparation kit can be used. Specifically, a liposome can be formed by dissolving and mixing lyophilized liposomes such as Lipocapsulater FD-S PE, FD-S MA, FD-S PL, FD-U PE, FD-U PL, and the like (commercially available from Katayama Chemical Industries) in a PBS buffer solution or the like at a concentration of 0.1 mg/40 µl to 10 mg/40 µl, and in this case, when the above peptide-binding lipid derivatives are mixed and heating is performed at a temperature equal to or higher than a phase transition temperature of the complex lipid, the peptide-binding lipid derivatives are incorporated into the liposome. Here, in this case, when an active component such as a drug, a nucleic acid (for example, single-stranded or double-stranded DNA or RNA), and a peptide is added and mixed, it is also possible to prepare a liposome encapsulating the active component.

### (Fusion of liposome with exosome)

Fusion of a liposome with an exosome can be performed using, for example, the method described in PTL 1, the content of which is incorporated as a part of this specification by reference. More specifically, a liposome into which peptide-binding lipid derivatives may be incorporated and an exosome may be mixed in the presence of polyethylene glycol (PEG). The amount of PEG added may be a concentration of about 0 mass% or more and 40 weight% or less in a mixed solution containing a liposome and an exosome. Here, examples of PEG to be used include PEG500 to PEG10000, and preferably PEG1000 to PEG8000.

### (Peptide-binding lipid derivatives)

When the exosome of the present embodiment is prepared as a hybrid liposome exosome, the hybrid liposome exosome may contain peptide-binding lipid derivatives in which a peptide binds to a hydrophilic part of a complex lipid in a lipid bilayer. When peptide-binding lipid derivatives are introduced into the hybrid liposome exosome, an ability of the hybrid liposome exosome to be incorporated into various cells can be improved without substantially influencing targeting of hybrid liposome exosomes. That is, the purpose of adding peptide-binding lipid derivatives is not to impart specific targeting to the hybrid liposome exosome, but to improve an ability of the hybrid liposome exosome to which predetermined targeting is imparted by overexpression of the integrin α, the integrin β, and their mutant proteins to be incorporated into the target cells.

Here, the method of forming a hybrid liposome exosome of the present invention is not particularly limited, but when peptide-binding lipid derivatives are added to the hybrid liposome exosome of the present embodiment, a liposome containing peptide-binding lipid derivatives is formed, this liposome is fused with an exosome, and thus a desired hybrid liposome exosome is obtained. Since the peptide-binding lipid derivatives are easily exposed on the outer surface of the hybrid liposome exosome, it is possible to impart an additional function related to incorporation into target cells to the hybrid liposome exosome. Therefore, it is possible to provide a carrier for a drug delivery system that can deliver an active component with a higher concentration to target cells.

### (Peptide that binds to peptide-binding lipid derivatives)

In the present embodiment, the peptide that binds to peptide-binding lipid derivatives preferably has 10 to 30 amino acid residues. When the number of amino acid residues of the peptide is within the above range, the function of the peptide that binds to peptide-binding lipid derivatives is sufficiently secured, and a bulky peptide is unlikely to inhibit fusion of the lipid bilayer of the hybrid liposome exosome and the lipid bilayer of the endosome. The peptide may have, for example, 10 to 27, 15 to 20, or 17 to 19 amino acid sequences.

When more specific description is provided in terms of these peptides, the above peptide is preferably a peptide that forms an α-helix structure with a hydrophobic surface in an endosome or a peptide having a basic residue.

### (Peptide that forms α-helix structure with hydrophobic surface)

A peptide that forms an α-helix structure with a hydrophobic surface in an endosome generally has a certain repeating sequence with a pitch of 3 to 4 residues as amino acid residues. Such a repeating sequence preferably contains a small bulk hydrophobic amino acid, a small bulk acidic amino acid and/or a polar amino acid. While the present invention is not bound by this theory, particularly, the acidic groups contained in the side chains of acidic amino acids have properties as weak acids, and thus the hybrid liposome exosome is incorporated into an endosome, and when the surrounding environment becomes acidic, the groups are protonated, and tend to form an α-helix structure with a hydrophobic surface together with other hydrophobic amino acid residues. When 10 to 30 amino acid residues having an a-helix structure with a hydrophobic surface are exposed on the outer surface of the lipid bilayer, they easily penetrate the lipid bilayer constituting an endosome (lysosome), and the hybrid liposome exosome and the endosome (lysosome) are more likely to be fused. That is, due to the action of the α-helix structure with a hydrophobic surface, the hybrid liposome exosome is more susceptible to endosome release, and the contents of the hybrid liposome exosome are more likely to be incorporated into the cytoplasm.

When the peptide is a peptide that forms an α-helix structure with a hydrophobic surface, the hydrophobic amino acid residue contained in the repeating sequence is preferably at least one selected from among an alanine residue, a leucine residue, and a methionine residue and more preferably at least one selected from among an alanine residue and a leucine residue. In addition, at least one residue of amino acid residues constituting the repeating sequence is preferably at least one selected from among an acidic amino acid residue and a polar amino acid residue described below. The acidic amino acid residue contained in the repeating sequence is preferably at least one selected from among an aspartic acid residue and a glutamic acid residue and more preferably a glutamic acid residue. The polar amino acid residue contained in the repeating sequence is preferably at least one selected from among an asparagine residue and a glutamine residue and more preferably a glutamine residue. Here, it is most preferable that the repeating sequence contain a combination of a hydrophobic amino acid residue and an acidic amino acid residue.

As an example of a peptide that satisfies such a condition, for example, an amino acid sequence having 30 residues shown in SEQ ID No. 1 (WEAALAEALAEALAEHLAEALAEALEALAA) may be exemplified.

### (Peptide having basic residue)

Examples of peptides having a basic residue include a peptide having at least one basic amino acid residue. When basic groups of the basic amino acid residues are protonated in the acidic environment in the endosome (lysosome), they easily electrostatically interact with hydrophilic parts such as phosphate groups that constitute the surface layer of the lipid bilayer. Therefore, incorporation of the hybrid liposome exosome into cells can be promoted. The peptide preferably has 2 to 5 consecutive basic amino acid residues, and more preferably 3 to 4 consecutive amino acid residues. When the above peptide has such consecutive basic amino acid residues, incorporation of the hybrid liposome exosome into cells can be further promoted.

In addition, the peptide preferably does not contain acidic amino acid residues. Thereby, the interaction between the basic amino acid residue in the peptide and the phosphate group on the surface layer of the lipid bilayer is not inhibited by the acidic amino acid residue, and incorporation of the hybrid liposome exosome into cells can be further promoted.

As an example of peptides that satisfy such conditions, for example, amino acid sequences shown in SEQ ID No. 2 (LLIILRRRIRKQAHAHSK), SEQ ID No. 3 (CGRKKRRQRRR), SEQ ID No. 4 (GIGAVLKVLTTGLPALISWIKRKRQQ), and SEQ ID No. 5 (RQIKIWFQNRRMKWKK) and having 18 residues, 11 residues, 26 residues, and 16 residues, respectively, may be exemplified.

Here, in the present embodiment, the amino acid residues constituting the peptide are not limited to amino acid residues derived from natural amino acids, and as long as effects of the present invention are not impaired, they may additionally include amino acid residues derived from amino acids other than natural amino acids and amino acid residues derived from amino acids, which are various isomers of natural amino acids such as optical isomers. Only 1 to 3 residues of various isomers of such unnatural amino acids or natural amino acids of the above peptide are preferably contained in the entire peptide, and all amino acid residues are more preferably amino acid residues derived from natural amino acids.

### (Complex lipid constituting peptide-binding lipid derivatives)

Examples of complex lipids constituting peptide-binding lipid derivatives include complex lipids known as biologically derived substances that are soluble in nonpolar solvents and constitute the lipid bilayer, and more specifically, complex lipids having, at an end, any reactive group selected from the group consisting of primary amino groups, carboxyl groups, hydroxy groups, phosphate groups, and thiol groups may be exemplified. These complex lipids are selected from among, for example, sphingophospholipids, glycosphingolipids, glycerophospholipids, glyceroglycolipids, ether type phospholipids, and ether type glycolipids. Since these complex lipids have polar groups derived from phosphate groups in their molecules, polar groups derived from sugars, and the like, such polar groups can react with polar groups of the peptide, and the complex lipid and the peptide can be easily bonded.

Examples of sphingophospholipids include sphingomyelin, and examples of glycosphingolipids include cerebroside (galactocerebroside, sulfatide, glucocerebroside, etc.), ganglioside, globoside, and sulfatide. In addition, examples of glycerophospholipids include phosphatidic acid, phosphatidylcholine, phosphatidylserine, phosphatidylethanolamine, and phosphatidylinositol, and examples of glyceroglycolipids include condensates of any monosaccharide or oligosaccharide and diacylglycerol such as monogalactosyldiacylglycerol. In addition, for example, saturated aliphatic groups and unsaturated aliphatic groups constituting hydrophobic groups of diacylglycerol moieties constituting glycerophospholipids and glyceroglycolipids are generally saturated aliphatic groups and unsaturated aliphatic groups derived from saturated or unsaturated fatty acids having 12 to 20 carbon atoms. Among these, examples of saturated fatty acids include lauric acid, myristic acid, pentadecylic acid, palmitic acid, heptadecanoic acid, stearic acid, and arachidic acid, and examples of unsaturated fatty acids include mono-unsaturated fatty acids (myristoleic acid, palmitoleic acid, sapienic acid, oleic acid, elaidic acid, vaccenic acid, gadoleic acid, eicosenoic acid, etc.), di-unsaturated fatty acids (linoleic acid, eicosadienoic acid, etc.), tri-unsaturated fatty acids (linolenic acid, pinolenic acid, etc.), tetra-unsaturated fatty acids (stearidonic acid, arachidonic acid, etc.), penta-unsaturated fatty acids (eicosapentaenoic acid, etc.), and hexa-unsaturated fatty acids (hexadocosaenoic acid, etc.). The aliphatic groups constituting the glycerophospholipid and glyceroglycolipid may include a mixture of saturated aliphatic groups and unsaturated aliphatic groups and a mixture of aliphatic groups having different numbers of carbon atoms and aliphatic groups having different numbers of unsaturated groups.

Ether type phospholipids and ether type glycolipids are complex lipids in which one or two long-chain hydrocarbon groups are ether-bonded to glycerol and which have a phosphate group or sugar as a polar group, and are widely found in thermophilic bacteria, and platelet activating factors and the like are known to have a similar structure. In this case, two hydrocarbon groups that bind to glycerol are preferably saturated or unsaturated hydrocarbon groups having 12 to 20 carbon atoms. That is, ether type phospholipids and ether type glycolipids are preferably those having a structure in which one or two molecules of a higher saturated or unsaturated alcohol having 12 to 20 carbon atoms and glycerol are bonded via an ether bond, and a phosphate group, or a monosaccharide or oligosaccharide is bonded to the remaining one hydroxyl group of glycerol.

Details of complex lipids are described in Int. J. Mol. Sci. 2019, 20, 2167, and Nat. Rev. Mol. Cell Biol., 2018, 19, 281, the contents of which are incorporated as a part of this specification by reference.

### (Method of forming peptide-binding lipid derivatives)

In the present embodiment, in order to form peptide-binding lipid derivatives, any chemical reaction method in which the N-terminal amino group or C-terminal carboxyl group of the peptide is covalently bonded to a phosphate group, amino group, hydroxyl group or the like of the complex lipid can be used. In the present embodiment, among such chemical reaction methods, a chemical reaction method in which a phosphate group, amino group, hydroxyl group or the like of a complex lipid is reacted with a carboxyl group or amino group introduced into the complex lipid via a linker to be described below and an amino group is preferably used. Examples of such chemical reaction methods include carbodiimide crosslinking reaction, NHS ester crosslinking reaction, and imidoester crosslinking reaction methods. In these chemical reaction methods, a carboxyl group or an amino group is activated with a predetermined compound and this is then covalently bonded to an amino group or a carboxyl group. Crosslinking agents for implementing these chemical reaction methods are commercially available from Thermo Fisher Scientific Inc., and those skilled in the art can use such commercially available products and implement the above chemical reaction method.

Examples of linkers for introducing a carboxyl group or amino group into a complex lipid include polyalkylene glycols such as polyethylene glycol and polypropylene glycol, and compounds in which a primary amino group or carboxyl group is introduced into these polyalkylene glycols. In this case, the polymerization degree of ethylene glycol or propylene glycol includes, for example, a polymerization degree of 20 or more and 50 or less, or 30 or more and 45 or less. More specific examples of linkers include compounds such as HOOC-PEG-COOH, H₂N-PEG-COOH, and H₂N-COOH-NH₂. These linkers are commercially available from Thermo Fisher Scientific Inc., and those skilled in the art can appropriately use such commercially available products. In addition, peptide-binding lipid derivatives may be formed using complex lipid derivatives obtained by introducing the crosslinking agent into the complex lipid (commercially available from Avanti Polar Lipids, Inc.) via the linker.

### [Method of detecting exosome and hybrid liposome exosome]

In the exosome and hybrid liposome exosome of the present embodiment formed by the method described above, when the exosome or hybrid liposome exosome to which at least one of antibodies selected from among antibodies conjugated with a first fluorescent dye, which specifically bind to an exosome marker protein selected from among CD63, CD81, and CD9, antibodies conjugated with a second fluorescent dye, which bind to an integrin α, antibodies conjugated with a third fluorescent dye, which bind to an integrin β, and antibodies conjugated with a fourth fluorescent dye, which bind to a protein disulfide isomerase (PDI) are added is analyzed using flow cytometry, at least one of antibodies selected from the group consisting of antibodies conjugated with a first fluorescent dye, antibodies conjugated with a second fluorescent dye, antibodies conjugated with a third fluorescent dye, and antibodies conjugated with a fourth fluorescent dye can be detected as vesicles bound together.

As these antibodies, those commercially available from Abcam and FUJIFILM Wako Pure Chemical Corporation can be used, and more specifically, products such as FITC Anti-Integrin alpha6, ab30496 (commercially available from Abcam), FITC Anti-Integrin Alpha beta3, ab93513 (commercially available from Abcam), Anti CD63, Monoclonal Antibodies (3-13), Fluorescein Conjugated, 018-2764 (commercially available from FUJIFILM Wako Pure Chemical Corporation), and ERp72(D70D12) XP Rabbit mAb (commercially available from Cell Signaling Technology) can be used. Here, regarding fluorescent dyes bound to these antibodies, commonly used fluorescent dyes may be exemplified as those bound to antibodies. Specific examples include Cy3, Cy5, FITC, TRITC, Rhodamine, TAMRA, Alexa Fluor, Texas Red, FAM, APC, PE, ATTO, and DyLight, but the present invention is not limited thereto.

### (Method of producing antibodies against peptides constituting peptide-binding lipid derivatives)

Here, antibodies against peptides constituting peptide-binding lipid derivatives can be produced using conventionally known methods. These antibodies may be monoclonal antibodies or polyclonal antibodies. In addition, these antibodies may be animal antibodies commonly used in production of antibodies such as mouse antibodies, rat antibodies, guinea pig antibodies, rabbit antibodies, and goat antibodies, or chimeric antibodies thereof. Hereinafter, a method of immunizing rabbits using synthetic peptides to acquire polyclonal antibodies will be exemplified.

### (Synthesis of peptide)

Peptides can be synthesized using methods that are commonly used for peptide synthesis, and are generally synthesized by a solid phase method, and the Boc method, Fmoc method and the like in which Boc, Fmoc and the like are used as a protecting group are commonly used. When peptides are synthesized by these methods, final deprotected peptide chains are purified and the purity thereof is checked by high performance liquid chromatography (HPLC), and the molecular weight thereof is checked by a mass analysis method, and thus it is checked whether they are appropriately synthesized and purified. The obtained peptides may be lyophilized and stored. Here, particularly, when short-chain peptides are produced as antigens, it is general to synthesize them using the chemical synthesis method, but when higher-molecular-weight proteins are used as antigens, for example, the proteins may be provided as antigens by overexpressing them in cultured cells or the like according to a general method, and purifying them using an appropriate combination of methods commonly used as peptide separation and purification methods such as SDS polyacrylamide gel electrophoresis (SDS-PAGE), gel filtration chromatography, HPLC, and affinity chromatography.

### (Rabbit immunization and antibody acquisition)

When the synthesized antigens are short-chain peptides, they are bonded to carrier proteins in order to improve immunogenicity thereof. Typical examples of carrier proteins include keyhole limpet hemocyanin (KLH) and bovine serum albumin (BSA), and the proteins can be bonded to the antigens using a commonly used method such as a maleimide method. Here, when immunogen proteins are polymers, binding to carrier proteins is not always necessary. These conjugated antigens or unconjugated antigens are mixed with an adjuvant and rabbits are immunized. As adjuvants, precipitating adjuvants (sodium hydroxide, aluminum hydroxide, calcium phosphate, aluminum phosphate, alum, PEPeS, carboxyvinyl polymer, etc.), oil-based adjuvants (liquid paraffin, lanolin, Freund, Freund's incomplete adjuvant, and Freund's complete adjuvant) and the like can be used.

When conjugated antigens or unconjugated antigens are administered to rabbits, 0.1 mg to 0.5 mg of the antigens is administered 4 to 6 times at 1- to 4-week intervals. Then, the antibody titer of the antiserum is checked, and when the antibody titer is 0.5 or more, whole blood is collected from the rabbit's carotid artery, and blood cell components are separated to obtain the antiserum. For this antiserum, a commonly used production method such as affinity chromatography using peptides or proteins as antigens is applied and monoclonal antibodies are obtained.

### (Fluorescent dye)

The polyclonal antibodies obtained as described above can be labeled with a fluorescent dye. Examples of such fluorescent dyes include commonly used fluorescent dyes with which antibodies are conjugated. Specific examples include Cy3, Cy5, FITC, TRITC, Rhodamine, TAMRA, Alexa Fluor, Texas Red, FAM, APC, PE, ATTO, and DyLight, but the present invention is not limited thereto.

### <Exosome-containing composition, and hybrid liposome exosome-containing composition>

The present embodiment also relates to an exosome-containing composition containing the exosome or hybrid liposome exosome of the present embodiment, and a hybrid liposome exosome-containing composition. Since the exosome and hybrid liposome exosome contained in such compositions have targeting to cells constituting specific tissues, when a certain disease is treated, such compositions can be used to deliver a drug (active component) effective in treating the disease to specific tissues.

Here, examples of such cells constituting specific tissues include cells constituting lung tissues and vascular endothelial cells, and particularly, when the specific tissues are lung tissues, examples of cells constituting lung tissues include bronchial epithelial cells, alveolar epithelial cells, pulmonary fibroblasts, and pulmonary smooth muscle cells. Examples of lung diseases to which the exosome-containing composition and hybrid liposome exosome-containing composition of the present embodiment can be applied include infectious lung diseases (for example, bronchitis, pneumonia), interstitial lung diseases (for example, interstitial pneumonia), emphysema (for example, chronic obstructive pulmonary disease (COPD)), lung tumors, and allergic lung diseases (for example, bronchial asthma, cough variant asthma). That is, the present embodiment also relates to an exosome-containing composition and a hybrid liposome exosome-containing composition, which contain an exosome or a hybrid liposome exosome, for delivering a therapeutically effective amount of an active component for treating the lung disease, alleviating symptoms and/or preventing the lung disease to lung tissues in order to treat the lung disease, alleviate symptom severity and/or prevent the lung disease. Here, treatment of lung diseases using the exosome-containing composition and the hybrid liposome exosome-containing composition containing the exosome or hybrid liposome exosome of the present embodiment can be equally applied to both human animals and non-human animals.

The composition of the present embodiment may include a pharmaceutically acceptable carrier. Such carriers can be appropriately selected by those skilled in the art according to the dosage form selected depending on the administration route of the composition.

### [Detection of exosome and hybrid liposome exosome by flow cytometry]

Here, in any process of the present invention, when exosomes and hybrid liposome exosomes are qualitatively or quantitatively detected, flow cytometry using antibodies labeled with a fluorescent dye can be used. When such flow cytometry is performed, the following points should be noted. (a) The antibody titer is determined using a microplate reader such as Synergy HTX (commercially available from BioTek Instruments) by measuring the change in absorbance due to color development by the enzyme using a conventionally known ELISA method (for example, refer to http://www.tkcraft.co.jp/page/elisa.htm). (b) Mouse antibodies are used as the first primary antibodies, and PE-CY7 labeled anti-mouse IgG goat antibodies are used as the first secondary antibodies. Rabbit antibodies are used as the second primary antibodies, and FITC labeled anti-rabbit IgG goat antibodies are used as the second secondary antibodies. (c) Regarding the amount of the first primary antibodies and the second primary antibodies added, within a range of 0.1 mg to 1 mg with respect to 100 µL of the exosome or hybrid liposome exosome-containing composition, the fluorescence intensity is measured by changing the addition amount, and the amount of both the first primary antibodies and the second primary antibodies added is determined by determining an addition amount at which a non-specific fluorescence is not observed under addition amount conditions in which a specific fluorescence changes depending on the addition amount. (d) As the flow cytometer, Attune (registered trademark) Acoustic Focusing Flow Cytometer (commercially available from Thermo Fisher Scientific Inc.) is used.

Regarding the above flow cytometry using magnetic beads, a PS Capture exosome flow cytometry kit (commercially available from FUJIFILM Wako Pure Chemical Corporation) can be used, and details of some protocols utilizing this kit are described in Nakai, W. et al., A novel affinity-based method for the isolation of highly purified extracellular vesicles. Sci. Rep. 6, 33935, the content of which is incorporated as a part of this specification by reference, but if there is any part that contradicts the description of a specific flow to be described below, the description of a specific flow to be described below has priority.

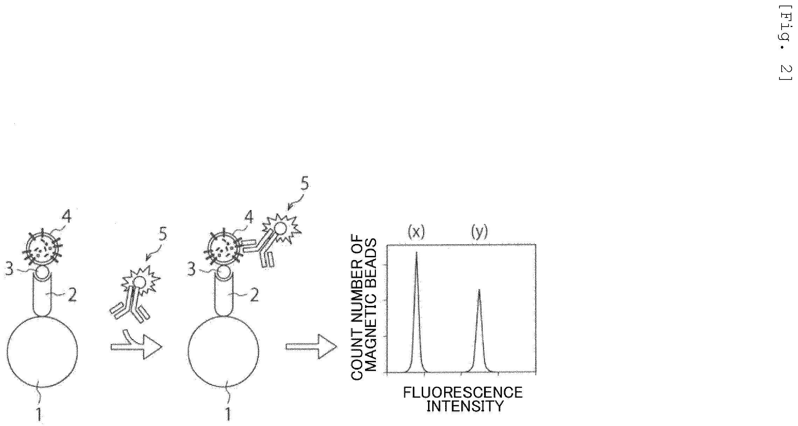

The principle of the PS Capture Exosome Flow Cytometry Kit is shown in Fig. 2 (a conceptual diagram of exosome flow cytometry using a phosphatidylserine-binding protein Tim4), and in this kit, a protein 2 (Tim4), which has an ability to bind to phosphatidylserine, binds to a magnetic bead 1, and this binds to a calcium ion 3-dependent exosome 4. When an exosome 5 is stained with a fluorescence-labeled antibody 5 specific to the exosome 4 bound to the magnetic bead 1, a flow cytometry peak (y) specific to the exosome 4 bound to the magnetic bead 1 is observed. Here, since binding of Tim4 to phosphatidylserine is dependent on the calcium ion 3, the calcium ion is removed using a chelating agent, and thus the exosome 4 is released from the magnetic bead 1.

More specifically, the following flow is used.

### (Separation of exosome or hybrid liposome exosome)

(A1) A supernatant is collected by centrifuging a cell culture supernatant at 300×g, 4°C, 5 minutes; 1,200×g, 4°C, 20 minutes; and 10,000×g, 4°C, 30 minutes.
(A2) 0.5 mL of Washing Buffer (10×) (included in the kit) and 4.5 mL of ultra-pure water are added to a 15 mL centrifuge tube and mixed with a vortex mixer. 50 µL (1/100 volume) of Exosome Binding Enhancer (included in the kit) is added thereto and mixed with a vortex mixer.
(A3) 300 µL of 2. is added to a new 1.5 mL microtube.
(A4) Exosome Capture Beads included in the PS Capture Exosome Flow Cytometry Kit are stirred with a vortex mixer for 10 seconds or longer.
(A5) 30 µL of Exosome Capture Beads are collected from (A4) and added to (3).
(A6) The 1.5 mL microtube of (A5) is vortexed for about 5 seconds, spin-downed in a desktop centrifuge and then set on a magnetic stand, and left for about 1 minute, and the supernatant is removed with a pipette.
(A7) 300 µL of (A2) is added to the 1.5 mL microtube of (A6) again, and (6) is repeated.
(A8) 100 µL of the supernatant of (A1) is added to the 1.5 mL microtube of (A7) and vortexed for about 5 seconds. 1 µL (1/100 volume) of Exosome Binding Enhancer is added thereto, and the mixture is vortexed for about 5 seconds.
(A9) (A8) is left at room temperature for 1 hour. During this time, the 1.5 mL microtube is vortexed for about 5 seconds at 20 minutes, 40 minutes, and 1 hour after a sample is added.
(A10) The 1.5 mL microtube of (A9) is spin-downed in a desktop centrifuge and then set on a magnetic stand, and left for about 1 minute, and the supernatant is removed with a pipette.
(A11) 300 µL of (A2) is added to the 1.5 mL microtube of (A10), and (A6) is repeated. This is repeated one more time.
(A12) 300 µL of (A2) is added to the 1.5 mL microtube of (A11) and vortexed for about 5 seconds.

### (Fluorescent antibodies staining and flow cytometry)

(B1) 100 µL of the exosome-containing composition or hybrid liposome exosome composition obtained in (A12) is dispensed into each of two 1.5 mL microtubes.
(B2) First primary antibodies and second primary antibodies are added in the addition amounts determined in the above (c) to the 1.5 mL microtube of (B1) and vortexed for about 3 seconds.
(B3) The 1.5 mL microtube of (B2) is left at room temperature for 1 hour. During this time, the 1.5 mL microtube is vortexed for about 5 seconds at 20 minutes, 40 minutes, and 1 hour after first primary antibodies and second primary antibodies are added.
(B4) The 1.5 mL microtube of (B3) is spin-downed in a desktop centrifuge and then set on a magnetic stand and left for about 1 minute, and the supernatant is removed with a pipette.
(B5) 300 µL of (A2) is added to the 1.5 mL microtube of (B4), and (B4) is repeated. This is repeated one more time.
(B6) (B2) to (B5) are repeated for first secondary antibodies and second secondary antibodies.
(B7) 300 µL of (A2) is added to the 1.5 mL microtube of (B6) and vortexed for about 5 seconds.
(B8) The sample of (B7) is used for flow cytometry. When flow cytometry is performed, fractions in which magnetic beads are not aggregated are gated based on a forward scattering and side scattering plot diagram, and the fluorescence intensity of magnetic beads contained in the gate is compared.

### [Examples]

### <Experimental Example 1-1; preparation of HEK293F cells for gene introduction>

HEK293F cells (derived from human embryonic kidney cells) that had been subcultured and maintained in advance in an HE100 medium (glutamine, penicillin-streptomycin solution formulation) (GMEP) in a 100 mm culture dish were detached from the culture surface by pipetting, and collected, and the number of cells was counted. The cells were seeded in a 6-well CellBIND (registered trademark, multi-well plate, commercially available from Corning (registered trademark)), and a 96-well CellBIND (registered trademark, multi-well plate, commercially available from Corning (registered trademark)) at 2×10⁵ cells/3 mL/well, and 1×10⁴ cells/100 µL/well, respectively, and cultured overnight under conditions of 37°C and 5% CO₂.

### <Experimental Example 1-2; introduction of genes into HEK293F cells-Lipofectamine method>

The medium was removed by suction from the cultured product of Experimental Example 1-1, and 100 µL of Opti-MEM (registered trademark, low serum medium, commercially available from Thermo Fisher Scientific) was added to each well of a 96-well plate and 5 mL of Opti-MEM was added to each well of a 6-well plate. Using Lipofectamine (registered trademark) LTX Reagent & (Plus Rreagent) (commercially available from Thermo Fisher Scientific), a DNA vector incorporating an integrin αv gene or an integrin α6 gene was added to the medium. In this case, 96 wells were adjusted so that the final concentration was 100 ng DNA/well, and 6 wells were adjusted so that the final concentration was 2.5 µg DNA/well. Here, seven isoforms of the integrin α6 were registered on the database, and sequences of three genes thereof were published. In the experimental example of the present invention, among these three genes, the gene encoding the longest protein (DB: UniProt, Accession No. P23229-2) was selected and used for subsequent experiments.

Here, more specific protocols for a method of introducing genes into HEK293 cells using the Lipofectamine method are described in Vida Mirzaie et al., "Enhancing the Butyrylcholinesterase Activity in HEK-293 Cell Line by Dual-Promoter Vector Decorated on Lipofectamine," Drug Design, Development and Therapy 2020:14 3589-3599, the contents of which are incorporated as a part of this specification by reference.

### <Experimental Example 1-3; collection of HEK293F cell-derived exosome>

The supernatant was removed from the cultured product of Experimental Example 1-2, and the medium was replaced with 5 mL of an HE100 medium. Culturing was performed for 16 days, and the supernatant was collected. Centrifugation was performed at 3,000 g×30 minutes at room temperature, and the supernatant was collected into a new 15 mL tube. The supernatant was stored at - 80°C until the exosomes were collected. The exosomes were collected from the centrifuged supernatant using MagCaptureExosome Isolation Kit PS Ver. 2 (commercially available from FUJIFILM Wako Pure Chemical Corporation) and a standard operation method (hereinafter sometimes referred to as "MagCapture method" in this specification) in the kit.

### <Experimental Example 1-4; examination of incorporating cells into exosome>

As model target cells for examining incorporation into exosomes, human umbilical vein endothelial cells (HUVEC) were used.

### [Examination of cell incorporation of integrin α gene-introduced HEK293F-derived exosome into HUVEC cells]

HUVEC cells that had been subcultured in advance in an Endothelial Cell Growth Medium 2 (endothelial cell growth medium, commercially available from PromoCell) in a T75 flask were detached from the plate surface by a trypsin method and collected, and the number of cells was counted. The cells were seeded in a 24-well plate at 5×10⁴ cells/500 µL/well, and cultured for 2 days under conditions of 37°C and 5% CO₂.

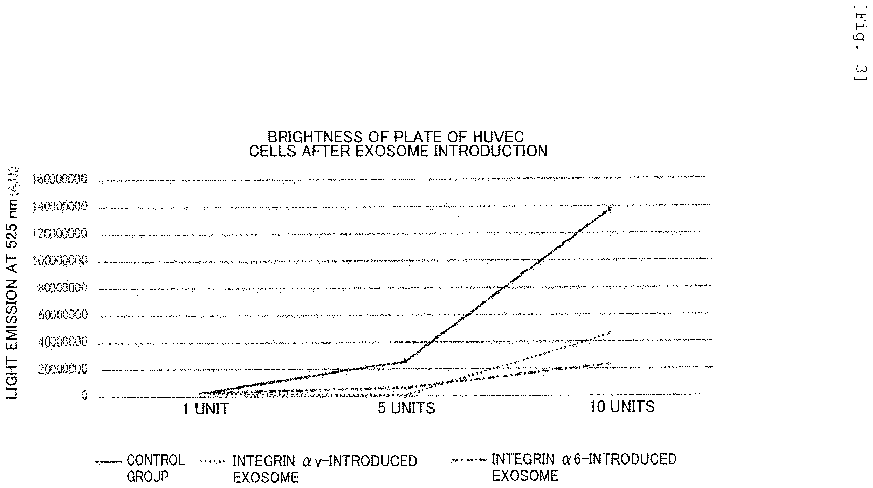

HEK293F cell-derived exosomes (prepared in Experimental Example 1-2) into which the integrin αv or integrin α6 was introduced were collected by the MagCapture method and suspended in 100 µL of PBS. These were subjected to green fluorescence staining according to the protocol of ExoSparkler Exosome Membrane Labeling Kit-Green (commercially available from Dojindo Molecular Technologies, Inc.). HUVEC cells were washed with 200 µL of PBS twice, and 500 µL of a new Endothelial Cell Growth Medium 2 was added. Fluorescently labeled exosomes were added to the medium, and culturing was performed for 4 hours under conditions of 37°C and 5% CO₂. After the sample medium was removed, washing with 200 µL of PBS was performed twice, and the HEK293F cell-derived exosomes in the supernatant were removed. The treated HUVEC cells were observed using an all-in-one fluorescence microscope BZ-X810 (commercially available from Keyence Corporation) at an excitation wavelength of 470/40 nm and a light emission wavelength of 525/50 nm, and the brightness (A.U.) of the plate was measured. The results are shown in Table 1 and Fig. 3. Here, in the drawing, 1 unit, 5 units, and 10 units indicate that the amount ratio of added exosomes was 1:5:10 between samples.

**[Table 2]**

| Table 1: Brightness of plate of HUVEC cells after exosome introduction | | | |
|---|---|---|---|
| | Control group | Integrin *α*v-introduced exosome | Integrin *α*6-introduced exosome |
| 1 unit | 2.5 × 10⁶ | 2.5 × 10⁶ | 3.3 × 10⁶ |
| 5 units | 2.6 × 10⁷ | 7.4 × 10⁵ | 5.9 × 10⁶ |
| 10 units | 1.4 × 10⁸ | 4.5 × 10⁷ | 2.4 × 10⁷ |

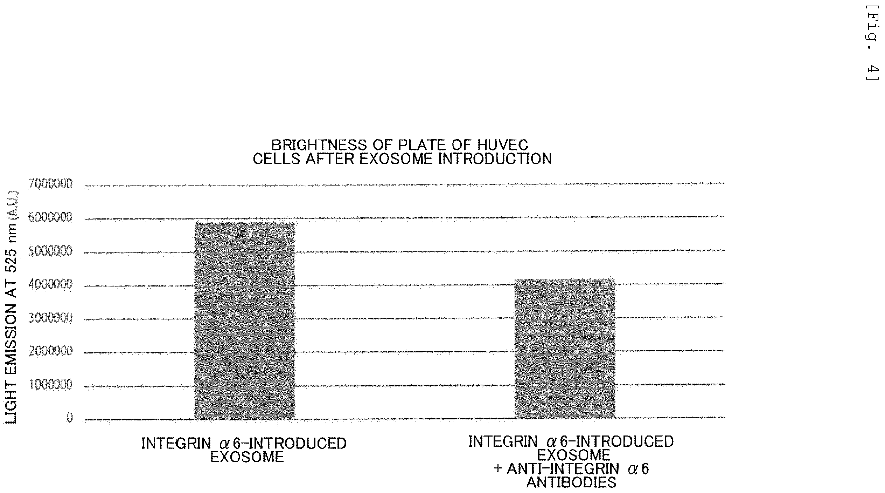

In the same manner as in [Examination of inhibition of cell incorporation of integrin α gene-introduced HEK293F-derived exosome into HUVEC cells using anti-integrin antibodies] and [Examination of cell incorporation of integrin α gene-introduced HEK293F-derived exosome into HUVEC cells], HUVEC cells were collected, and the cells were seeded in a 24-well plate at 5×10⁴ cells/500 µL/well, and cultured for 2 days under conditions of 37°C and 5% CO₂. Exosomes (prepared in Experimental Example 1-2) bound to magnetic beads were collected by the MagCapture method, 10 µL of a 10% bovine serum albumin (BSA) was then added, the mixture was incubated at 4°C for 30 minutes, and blocking was performed. Then, the BSA solution was removed and washed using a predetermined method (MagCapture method), and 1 µL of anti-integrin α6 antibodies (27189-1-AP, commercially available from Proteintech) was added thereto and the mixture was incubated at 4°C for 1 hour. Then, the antibody solution was removed and washed using a predetermined method (MagCapture method), and the exosomes were separated from the magnetic beads and collected. HEK293F cell-derived exosomes into which the integrin αv or integrin α6 was introduced were suspended in 100 µL of PBS. These were subjected to green fluorescence staining according to the protocol of ExoSparkler Exosome Membrane Labeling Kit-Green (commercially available from Dojindo Molecular Technologies, Inc.). HUVEC cells were washed with 200 µL of PBS twice, and 500 µL of a new Endothelial Cell Growth Medium 2 was added. Fluorescently labeled exosomes were added to the medium, and culturing was performed for 4 hours under conditions of 37°C and 5% CO₂. After the sample medium was removed, washing with 200 µL of PBS was performed twice, and the HEK293F cell-derived exosomes in the supernatant were removed. The treated HUVEC cells were observed using an all-in-one fluorescence microscope BZ-X810 (commercially available from Keyence Corporation) at an excitation wavelength of 470/40 nm and a light emission wavelength of 525/50 nm, and the brightness (A.U.) of the plate was measured. The results are shown in Table 2 and Fig. 4.

**[Table 3]**

| Table 2: Brightness of plate of HUVEC cells after exosome introduction | |
|---|---|
| | Brightness (integration, A.U.) |
| Integrin *α*6-introduced exosome | 5.9 × 10⁶ |
| Integrin *α*6-introduced exosome + anti-integrin *α*6 antibodies | 4.2 × 10⁶ |

As shown in Fig. 3, exosomes into which the integrin αv or integrin α6 was introduced were incorporated into vascular endothelial cells, but the percentage thereof was lower than that of control group exosomes using the same amount of exosomes. On the other hand, as shown in Fig. 4, in exosomes in which the function of the integrin α6 was inhibited using anti-integrin α6 antibodies, incorporation into vascular endothelial cells disappeared almost completely. These facts indicate that, when the integrin αv and the integrin α6 were introduced into exosomes, targeting to vascular endothelial cells was maintained, but for some reasons, the incorporation amount itself tended to decrease.

### <Experimental Example 1-5; preparation of peptide-added hybrid liposome exosome>

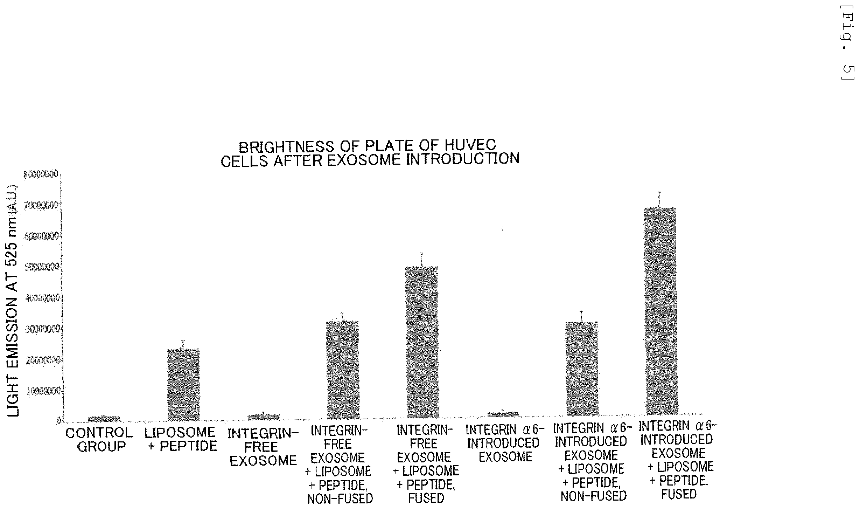

FD-U PL (1,2-dioleyl-sn-glycero-3-phosphocholine (DOPC):cholesterol=70:30) was weighed out in a 1.5 mL tube and suspended in PBS(-). Peptides fluorescently labeled with FITC were collected in a 1.5 mL tube and dissolved in PBS. This peptide solution and DSPE-PEG2000-NHS solution (DSPE; distearylphosphatidylethanolamine) were mixed and left at room temperature for 2 hours, and thus NHS was reacted with amino groups of the peptides. In addition, the DSPE-PEG2000-NHS-peptide solution was mixed with a liposome solution. The mixed solution containing FD-U PL and DSPE-PEG2000-NHS-peptides was heated at 37°C for 30 minutes. Here, for the peptides, those obtained by binding FITC to the C-terminal of the peptide shown in SEQ ID No. 3 and those obtained by binding FIPTC to the C-terminal of the peptide shown in SEQ ID No. 5 were separately used. The peptide-added liposome and the exosome solution obtained from cells in which the integrin α was overexpressed, which was obtained in Experimental Example 1-4, were mixed. In addition, a PEG-8000 solution was added so that the concentration was 30%. The mixture was left at 40°C for 2 hours, and the liposome and the exosome were fused. As a PEG solution control, a PBS(-) solution was used (indicated as "non-fused"). The obtained hybrid liposome exosome was subjected to an experiment of incorporation into HUVEC cells according to the protocol described in Experimental Example 1-4. The treated HUVEC cells were observed using an all-in-one fluorescence microscope BZ-X810 (commercially available from Keyence Corporation) at an excitation wavelength of 470/40 nm and a light emission wavelength of 525/50 nm, and the brightness (A. U.) of the plate was measured. The results are shown in Table 3 and Fig. 5. Here, when the peptide of SEQ ID No. 5 was used in place of the peptide of SEQ ID No. 3, generally, the results showed similar trends to those shown in Table 3 and Fig. 5.

**[Table 4]**

| Table 3: Brightness of plate of HUVEC cells after exosome introduction | | |
|---|---|---|
| | Number of data items | Brightness (average, A.U.) |
| Control group | 4 | 1.7 × 10⁶ |
| Liposome + peptide | 4 | 2.3 × 10⁷ |
| Integrin-free exosome | 4 | 1.7 × 10⁶ |
| Integrin-free exosome + liposome + peptide, non-fused | 4 | 3.2 × 10⁷ |
| Integrin-free exosome + liposome + peptide, fused | 4 | 4.9 × 10⁷ |
| Integrin *α*6-introduced exosome | 4 | 1.4 × 10⁶ |
| Integrin *α*6-introduced exosome + liposome + peptide, non-fused | 4 | 3.0 × 10⁷ |
| Integrin *α*6-introduced exosome + liposome + peptide, fused | 4 | 6.7 × 10⁷ |

As can be clearly understood from Table 3 and Fig. 5, when the exosome into which the integrin α6 was introduced was fused with the liposome retaining peptides, the result of incorporation into HUVEC cells was improved by 120% or more, compared to the mixture of corresponding non-fused exosomes and liposomes.

### <Experimental Example 2-1; introduction of genes into HEK293F cells-Lipofectamine method>

The medium was removed by suction from the cultured product of Experimental Example 1-1, and 100 µL of Opti-MEM (registered trademark, low serum medium, commercially available from Thermo Fisher Scientific) was added to each well of a 96-well plate and 5 mL of Opti-MEM was added to each well of a 6-well plate. Using Lipofectamine (registered trademark) LTX Reagent & (Plus Rreagent) (commercially available from Thermo Fisher Scientific), a DNA vector incorporating an integrin β4 gene, an integrin β5 gene, or an integrin β6 gene was added to the medium. In this case, 96 wells were adjusted so that the final concentration was 100 ng DNA/well, and 6 wells were adjusted so that the final concentration was 2.5 µg DNA/well.

### <Experimental Example 2-2; collection of HEK293F cell-derived exosome>

The supernatant was removed from the cultured product of Experimental Example 2-1, and the medium was replaced with 5 mL of an HE100 medium. Culturing was performed for 16 days, and the supernatant was collected. Centrifugation was performed at 3,000 g×30 minutes at room temperature, and the supernatant was collected into a new 15 mL tube. The supernatant was stored at - 80°C until the exosomes were collected. The exosomes were collected from the centrifuged supernatant using the MagCapture method.

### <Experimental Example 2-3; examination of incorporating cells into exosome>

Human pulmonary fibroblasts (HPF) were used as model target cells for examining incorporation into exosomes.

### [Examination of cell incorporation of integrin α gene- or integrin β gene-introduced HEK293F cell-derived exosome into HPF cells]

HPF cells that had been subcultured and maintained in advance in a fibroblast growth medium 2 (model number: D12042, commercially available from PromoCell) in a T75 flask were detached from the plate surface by a trypsin method and collected, and the number of cells was counted. The cells were seeded in 24 wells at 5×10⁴ cells/500 µL/well, and cultured overnight under conditions of 37°C and 5% CO₂.

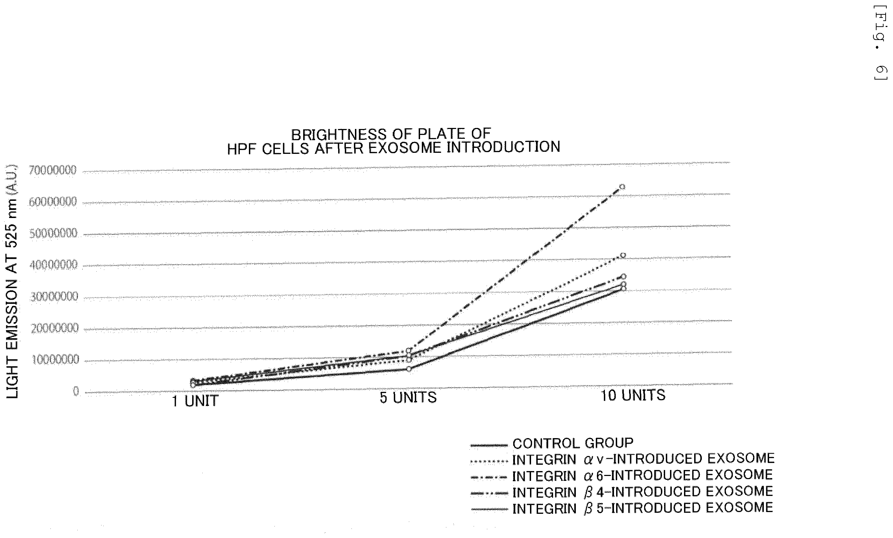

HEK293F cell-derived exosomes into which the integrin α gene or integrin β gene was introduced were collected by the MagCapture method and suspended in 100 µL of PBS. Green fluorescence staining was performed according to the protocol of ExoSparkler Exosome Membrane Labeling Kit-Green (commercially available from Dojindo Molecular Technologies, Inc.). HPF cells were washed with 200 µL of PBS twice, and 500 µL of a new fibroblast growth medium 2 (model number: D12042, commercially available from PromoCell) was added. Fluorescently labeled exosomes were added to the medium, and culturing was performed for 4 hours under conditions of 37°C and 5% CO₂. After the sample medium was removed, washing with 200 µL of PBS was performed twice, and the HEK293F cell-derived exosomes in the supernatant were removed. The treated HPF cells were observed using an all-in-one fluorescence microscope BZ-X810 (commercially available from Keyence Corporation) at an excitation wavelength of 470/40 nm and a light emission wavelength of 525/50 nm, and the brightness (A. U.) of the plate was measured. The results are shown in Table 4 and Fig. 6. Here, in the drawing, 1 unit, 5 units, and 10 units indicate that the amount ratio of added exosomes was 1:5:10 between samples.

**[Table 5]**

| Table 4: Brightness of plate of HPF cells after exosome introduction | | | | | |
|---|---|---|---|---|---|
| | Control group | Integrin *α*v-introduced exosome | Integrin *α*6-introduced exosome | Integrin *β*4-introduced exosome | Integrin *β*5-introduced exosome |
| 1 unit | 3.1 × 10⁶ | 2.7 × 10⁶ | 3.3 × 10⁶ | 1.9 × 10⁶ | 1.9 × 10⁶ |
| 5 units | 1.0 × 10⁷ | 9.0 × 10⁶ | 1.2 × 10⁷ | 1.0 × 10⁷ | 6.1 × 10⁶ |
| 10 units | 3.2 × 10⁷ | 4.1 × 10⁷ | 6.3 × 10⁷ | 3.5 × 10⁷ | 3.1 × 10⁷ |

### [Examination of inhibition of incorporation of integrin α gene- or integrin β gene-introduced HEK293F-derived exosome into HPF cells using anti-integrin antibodies]

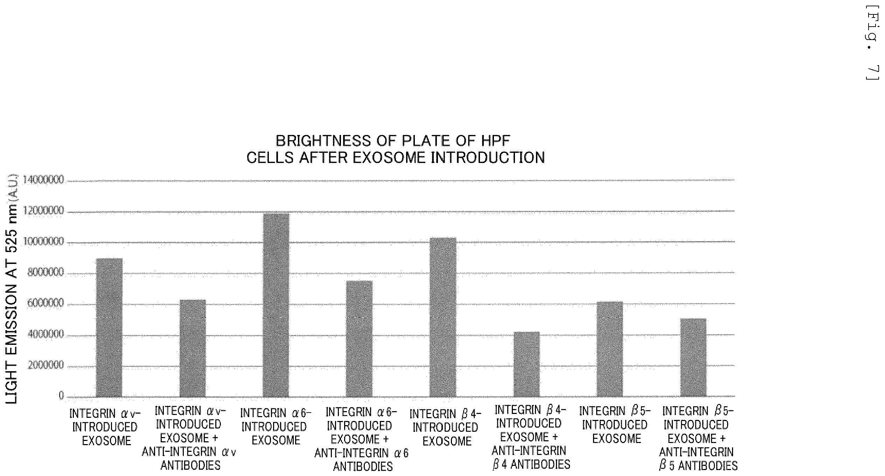

In the same manner as in [Examination of cell incorporation of integrin α gene- or integrin β gene-introduced HEK293F cell-derived exosome into HPF cells], HPF cells were collected, the cells were seeded in 24 wells at 5×10⁴ cells/500 µL/well, and cultured overnight under conditions of 37°C and 5% CO₂. Exosomes (prepared in Experimental Example 2-1) bound to magnetic beads were collected by the MagCapture method, 10 µL of a 10% bovine serum albumin (BSA) was then added, the mixture was incubated at 4°C for 30 minutes, and blocking was performed. Then, the BSA solution was removed and washed using a predetermined method (MagCapture method), and 1 µL of anti-integrin αv antibodies (model number 27096-1-AP, commercially available from Proteintech), anti-integrin α6 antibodies (27189-1-AP, commercially available from Proteintech), anti-integrin β4 antibodies (model number 21738-1-AP, commercially available from Proteintech), and anti-integrin β5 antibodies (model number D24A5, rabbit monoclonal antibodies #3629, commercially available from Cell Signaling Technology) was added thereto, and the mixture was incubated at 4°C for 1 hour. Then, the antibody solution was removed and washed using a predetermined method (MagCapture method), and the exosomes were separated from the magnetic beads and collected. HEK293F cell-derived exosomes (prepared in Experimental Example 2-1) into which the integrin αv, the integrin α6, the integrin β4, or the integrin β5 was introduced were suspended in 100 µL of PBS. These were subjected to green fluorescence staining according to the protocol of ExoSparkler Exosome Membrane Labeling Kit-Green (commercially available from Dojindo Molecular Technologies, Inc.). HUVEC cells were washed with 200 µL of PBS twice, and 500 µL of a new Endothelial Cell Growth Medium 2 was added. Fluorescently labeled exosomes were added to the medium, and culturing was performed for 4 hours under conditions of 37°C and 5% CO₂. After the sample medium was removed, washing with 200 µL of PBS was performed twice, and the HEK293F cell-derived exosomes in the supernatant were removed. The treated HUVEC cells were observed using an all-in-one fluorescence microscope BZ-X810 (commercially available from Keyence Corporation) at an excitation wavelength of 470/40 nm and a light emission wavelength of 525/50 nm, and the brightness (A. U.) of the plate was measured. The results are shown in Table 5 and Fig. 7.

**[Table 6]**

| Table 5: Brightness of plate of HPF cells after exosome introduction | |
|---|---|
| | Brightness (integration, A.U.) |
| Integrin *α*v-introduced exosome | 9.0 × 10⁷ |
| Integrin *α*v-introduced exosome + anti-integrin *α*v antibodies | 6.3 × 10⁶ |
| Integrin *α*6-introduced exosome | 1.2 × 10⁷ |
| Integrin *α*6-introduced exosome + anti-integrin *α*6 antibodies | 1.5 × 10⁶ |
| Integrin *β*4-introduced exosome | 1.0 × 10⁷ |
| Integrin *β*4-introduced exosome + anti-integrin *β*4 antibodies | 4.2 × 10⁶ |
| Integrin *β*5-introduced exosome | 6.1 × 10⁶ |
| Integrin *β*5-introduced exosome + anti-integrin *β*5 antibodies | 5.1 × 10⁶ |

As shown in Table 4 and Fig. 6, exosomes into which the integrin αv, the integrin α6, the integrin β4, or the integrin β5 was introduced were incorporated into pulmonary fibroblasts, but the percentage thereof was lower than that of control group exosomes using the same amount of exosomes. On the other hand, as shown in Table 5 and Fig. 7, in exosomes in which the function of the integrin α or integrin β was inhibited using anti-integrin αv antibodies, anti-integrin α6 antibodies, anti-integrin β4 antibodies, or anti-integrin β5 antibodies, incorporation into pulmonary fibroblasts was reduced to about 40% to 85%. These facts indicate that, when at least the integrin β4 or integrin β5 was introduced into exosomes, targeting to pulmonary fibroblasts was maintained, but for some reasons, the incorporation amount itself tended to decrease.

### <Experimental Example 2-4; comparison of incorporation of exosome into HPF cells and HUVEC cells>

HPF cells and human umbilical vein endothelial cells (HUVEC) were used as model target cells for examining incorporation into exosomes. HPF cells and exosomes were prepared in the same manner as in [Examination of cell incorporation of integrin α gene- or integrin β gene-introduced HEK293F cell-derived exosome into HPF cells] in Experimental Example 2-3. The method of preparing HUVEC cells is shown below.

### [Preparation of HUVEC cells]

HUVEC cells that had been subcultured in advance in an Endothelial Cell Growth Medium 2 (endothelial cell growth medium, commercially available from PromoCell) in a T75 flask were detached from the plate surface by a trypsin method and collected, and the number of cells was counted. The cells were seeded in a 24-well plate at 5×10⁴ cells/500 µL/well, and cultured for 2 days under conditions of 37°C and 5% CO₂.

### [Cell incorporation of integrin α gene- or integrin β gene-introduced HEK293F cell-derived exosome into HUVEC cells]

HEK293F cell-derived exosomes into which the above integrin α gene and/or integrin β gene was introduced were suspended in 100 µL of PBS. Green fluorescence staining was performed according to the protocol of ExoSparkler Exosome Membrane Labeling Kit-Green (commercially available from Dojindo Molecular Technologies, Inc.). HUVEC cells were washed with 200 µL of PBS twice, and 500 µL of a new Endothelial Cell Growth Medium 2 (endothelial cell growth medium, commercially available from PromoCell) was added. Fluorescently labeled exosomes were added to the medium, and culturing was performed for 4 hours under conditions of 37°C and 5% CO₂.

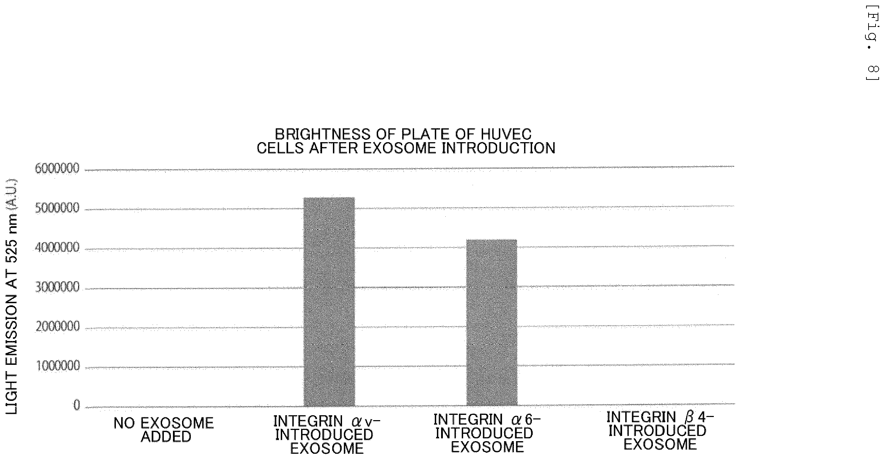

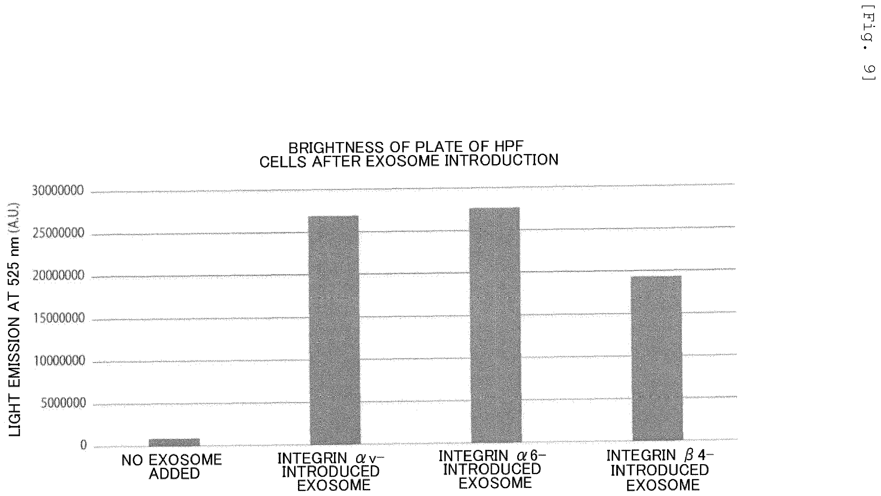

Using HPF cells and HUVEC cells, in the same manner as in [Examination of cell incorporation of integrin α gene- or integrin β gene-introduced HEK293F cell-derived exosome into HPF cells] in Experimental Example 2-3, exosomes in which the integrin αv, the integrin α6, or the integrin β4 was expressed were added. The treated HPF cells and HUVEC cells were observed using an all-in-one fluorescence microscope BZ-X810 (commercially available from Keyence Corporation) at an excitation wavelength of 470/40 nm and a light emission wavelength of 525/50 nm, and the brightness (A.U.) of the plate was measured. The results are shown in Table 6 and Fig. 8 and Fig. 9.

**[Table 7]**

| Table 6: Brightness of plate of HUVEC cells after exosome introduction | |
|---|---|
| | Brightness (A.U.) |
| No exosome added | 3.2 × 10³ |
| Integrin *α*v-introduced exosome | 5.3 × 10⁶ |
| Integrin *α*6-introduced exosome | 4.2 × 10⁶ |
| Integrin *β*4-introduced exosome | 3.9 × 10³ |

As can be clearly understood from Table 6 and Fig. 8, the exosome into which the integrin αv and the integrin α6 were introduced was specifically incorporated into both HPF cells and HUVEC cells. On the other hand, as shown in Table 6 and Fig. 9, the exosome into which the integrin β4 was introduced was specifically incorporated into HPF cells, but was hardly incorporated into HUVEC cells.

### <Experimental Example 2-5; preparation of peptide-added hybrid liposome exosome>

HPF cells and HUVEC cells were used as model target cells for examining incorporation into hybrid liposome exosomes.

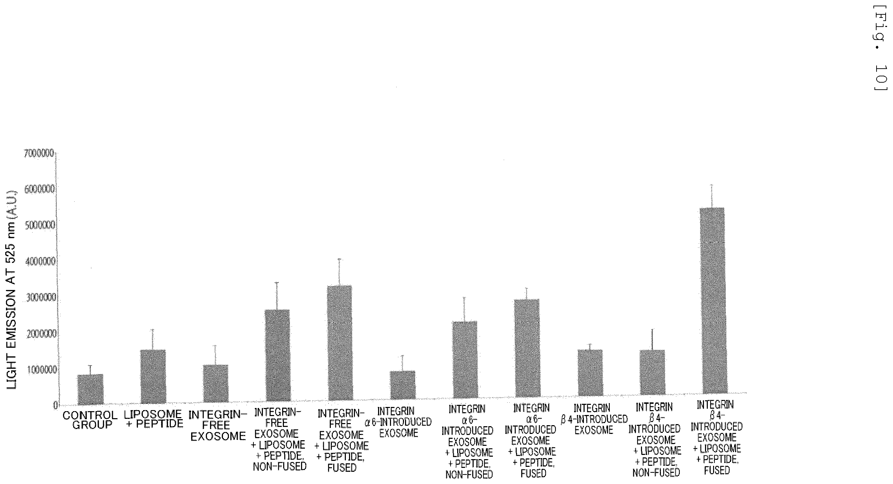

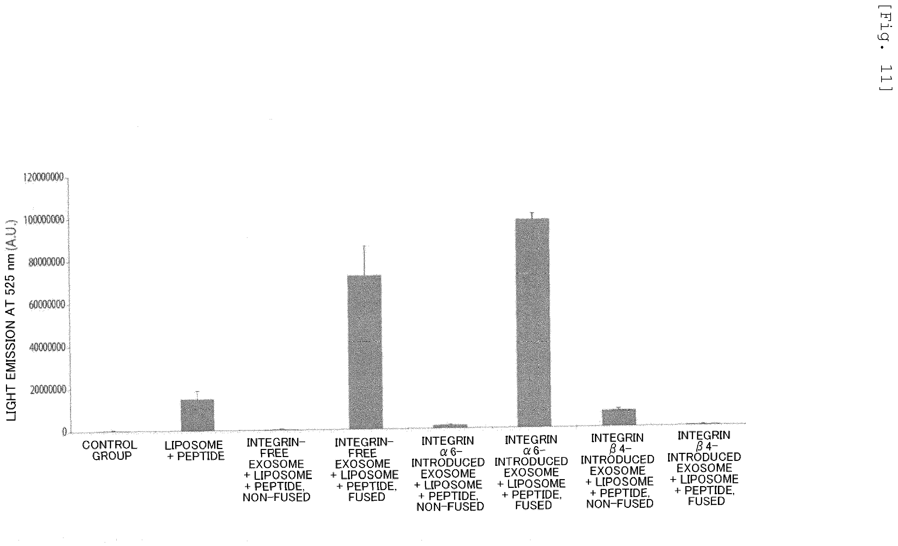

FD-U PL (1,2-dioleyl-sn-glycero-3-phosphocholine (DOPC):cholesterol=70:30) was weighed out in a 1.5 mL tube and suspended in PBS(-). Peptides fluorescently labeled with FITC were collected in a 1.5 mL tube and dissolved in PBS. This peptide solution and DSPE-PEG2000-NHS solution (DSPE; distearylphosphatidylethanolamine) were mixed and left at room temperature for 2 hours, and thus NHS was reacted with amino groups of the peptides. In addition, the DSPE-PEG2000-NHS-peptide solution was mixed with a liposome solution. The mixed solution containing FD-U PL and DSPE-PEG2000-NHS-peptides was heated at 37°C for 30 minutes. Here, for the peptides, those obtained by binding FITC to the C-terminal of the peptide shown in SEQ ID No. 3 and those obtained by binding FIPTC to the C-terminal of the peptide shown in SEQ ID No. 5 were separately used. The peptide-added liposome and the exosome solution obtained from cells in which the integrin α6 or integrin β4 was overexpressed were mixed. In addition, a PEG-8000 solution was added so that the concentration was 30%. The mixture was left at 40°C for 2 hours, and the liposome and the exosome were fused. As a PEG solution control, a PBS(-) solution was used (indicated as "non-fused"). The obtained hybrid liposome exosome was subjected to an experiment of incorporation into HUVEC cells and HPF cells according to the protocol described in Experimental Example 2-3. The treated HUVEC cells were observed using an all-in-one fluorescence microscope BZ-X810 (commercially available from Keyence Corporation) at an excitation wavelength of 470/40 nm and a light emission wavelength of 525/50 nm, and the brightness (A.U.) of the plate was measured. The results are shown in Tables 7 and 8, and Figs. 10 and 11. Here, when the peptide of SEQ ID No. 5 was used in place of the peptide of SEQ ID No. 3, generally, the results showed similar trends to those shown in Table 7 and Table 8, and Fig. 10 and Fig. 11.

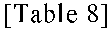

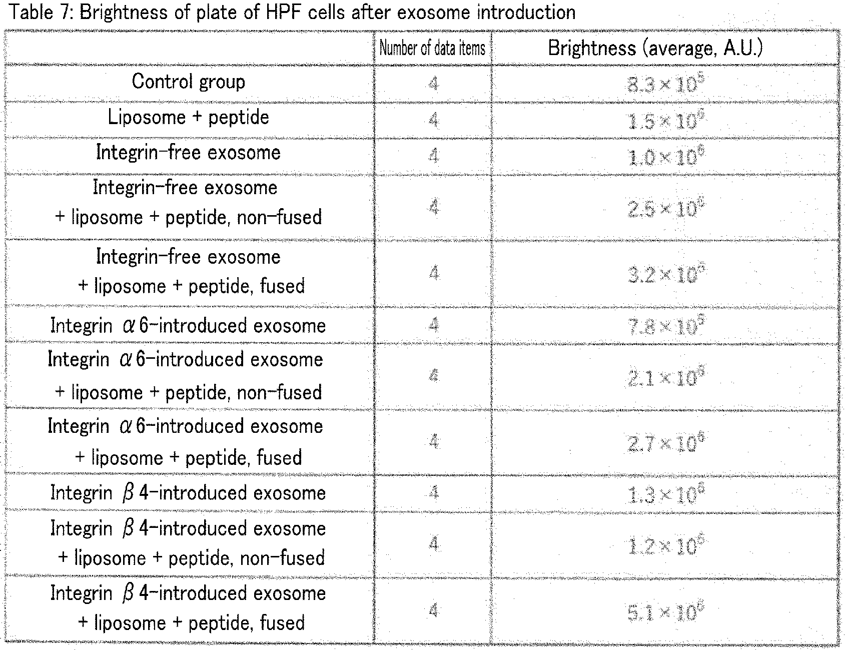

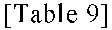

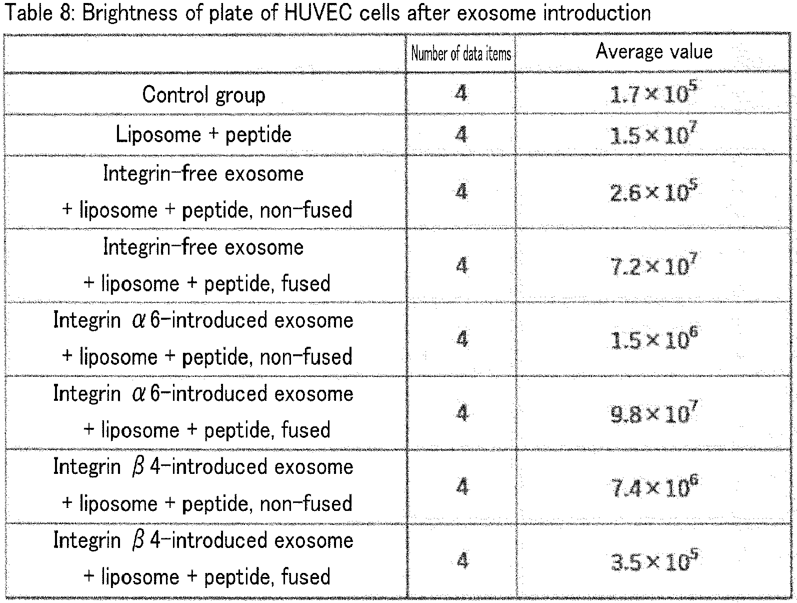

As can be clearly understood from Table 7 and Table 8, and Fig. 10 and Fig. 11, when the exosome into which the integrin β4 was introduced was fused with the liposome retaining peptides, the result of incorporation into HPF cells was improved by 300% or more, compared to the mixture of corresponding non-fused exosomes and liposomes.

### <Experimental Example 3-1; introduction of genes into HEK293F cells-Lipofectamine method>

The medium was removed by suction from the cultured product of Experimental Example 1-1, and 100 µL of Opti-MEM (registered trademark, low serum medium, commercially available from Thermo Fisher Scientific) was added to each well of a 96-well plate and 5 mL of Opti-MEM was added to each well of a 6-well plate. Using Lipofectamine (registered trademark) LTX Reagent & (Plus Rreagent) (commercially available from Thermo Fisher Scientific), a DNA vector incorporating an integrin αv gene, an integrin α6 gene, or an integrin β4 gene was added to the medium. Here, when the integrin α6 gene and the integrin β4 gene were introduced at the same time, the two genes were incorporated into the same vector under control of CMV promoters. In this case, 96 wells were adjusted so that the final concentration was 100 ng DNA/well, and 6 wells were adjusted so that the final concentration was 2.5 µg DNA/well.

### <Experimental Example 3-2; collection of HEK293F cell-derived exosome>

The supernatant was removed from the cultured product of Experimental Example 3-1, and the medium was replaced with 5 mL of an HE100 medium. Culturing was performed for 16 days, and the supernatant was collected. Centrifugation was performed at 3,000 g×30 minutes at room temperature, and the supernatant was collected into a new 15 mL tube. The supernatant was stored at - 80°C until the exosomes were collected. The exosomes were collected from the centrifuged supernatant using the MagCapture method.

### <Experimental Example 3-3; examination of incorporating cells into exosome>

Human pulmonary fibroblasts (HPF) were used as model target cells for examining incorporation into exosomes.

### [Examination of cell incorporation of integrin α gene- or integrin β gene-introduced HEK293F cell-derived exosome into HPF cells]

HPF cells that had been subcultured and maintained in advance in a fibroblast growth medium 2 (model number: D12042, commercially available from PromoCell) in a T75 flask were detached from the plate surface by a trypsin method and collected, and the number of cells was counted. The cells were seeded in 24 wells at 5×10⁴ cells/500 µL/well, and cultured overnight under conditions of 37°C and 5% CO₂.

HEK293F cell-derived exosomes into which the integrin α gene or integrin β gene was introduced were collected by the MagCapture method and suspended in 100 µL of PBS. Green fluorescence staining was performed according to the protocol of ExoSparkler Exosome Membrane Labeling Kit-Green (commercially available from Dojindo Molecular Technologies, Inc.). HPF cells were washed with 200 µL of PBS twice, and 500 µL of a new fibroblast growth medium 2 (model number: D12042, commercially available from PromoCell) was added. Fluorescently labeled exosomes were added to the medium, and culturing was performed for 4 hours under conditions of 37°C and 5% CO₂. After the sample medium was removed, washing with 200 µL of PBS was performed twice, and the HEK293F cell-derived exosomes in the supernatant were removed. The treated HPF cells were observed using an all-in-one fluorescence microscope BZ-X810 (commercially available from Keyence Corporation) at an excitation wavelength of 470/40 nm and a light emission wavelength of 525/50 nm, and the brightness (A. U.) of the plate was measured.

### [Examination of inhibition of incorporation of integrin α gene- or integrin β gene-introduced HEK293F-derived exosome into HPF cells using anti-integrin antibodies]

In the same manner as in [Examination of cell incorporation of integrin α gene- or integrin β gene-introduced HEK293F cell-derived exosome into HPF cells], HPF cells were collected, the cells were seeded in 24 wells at 5×10⁴ cells/500 µL/well, and cultured overnight under conditions of 37°C and 5% CO₂. Exosomes (prepared in Experimental Example 3-1) bound to magnetic beads were collected by the MagCapture method, 10 µL of a 10% bovine serum albumin (BSA) was then added, the mixture was incubated at 4°C for 30 minutes, and blocking was performed. Then, the BSA solution was removed and washed using a predetermined method (MagCapture method), and 1 µL of anti-integrin αv antibodies (model number 27096-1-AP, commercially available from Proteintech), anti-integrin α6 antibodies (27189-1-AP, commercially available from Proteintech), and anti-integrin β4 antibodies (model number 21738-1-AP, commercially available from Proteintech) was added thereto, and the mixture was incubated at 4°C for 1 hour. Then, the antibody solution was removed and washed using a predetermined method (MagCapture method), and the exosomes were separated from the magnetic beads and collected. HEK293F cell-derived exosomes into which the integrin αv, integrin α6, or integrin β4 was introduced were suspended in 100 µL of PBS. These were subjected to green fluorescence staining according to the protocol of ExoSparkler Exosome Membrane Labeling Kit-Green (commercially available from Dojindo Molecular Technologies, Inc.). HPF cells were washed with 200 µL of PBS twice, and 500 µL of a new fibroblast growth medium 2 (model number: D12042, commercially available from PromoCell) was added. Fluorescently labeled exosomes were added to the medium, and culturing was performed for 4 hours under conditions of 37°C and 5% CO₂. After the sample medium was removed, washing with 200 µL of PBS was performed twice, and the HEK293F cell-derived exosomes in the supernatant were removed. The treated HPF cells were observed using an all-in-one fluorescence microscope BZ-X810 (commercially available from Keyence Corporation) at an excitation wavelength of 470/40 nm and a light emission wavelength of 525/50 nm, and the brightness (A.U.) of the plate was measured.

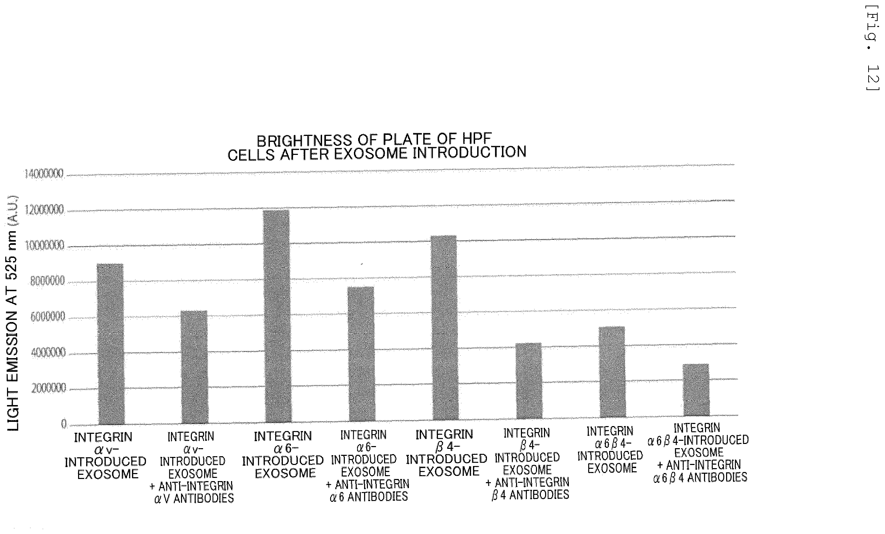

The above results are shown in Table 9 and Fig. 12.

**[Table 10]**

| Table 9: Brightness of plate of HPF cells after exosome introduction | |
|---|---|
| | Brightness (integration, A.U.) |
| Integrin *α*v-introduced exosome | 9.0 × 10⁶ |
| Integrin *α*v-introduced exosome + anti-integrin *α*v antibodies | 6.3 × 10³ |
| Integrin *α*6-introduced exosome | 1.2 × 10⁷ |
| Integrin *α*6-introduced exosome + anti-integrin *α*6 antibodies | 7.5 × 10⁶ |
| Integrin *β*4-introduced exosome | 1.0 × 10⁷ |
| Integrin *β*4-introduced exosome + anti-integrin *β*4 antibodies | 4.2 × 10⁶ |
| Integrin *α*6*β*4-introduced exosome | 5.1 × 10⁶ |
| Integrin *α*6*β*4-introduced exosome + anti-integrin *α*6*β*4 antibodies | 2.9 × 10⁶ |

As shown in Table 9 and Fig. 12, exosomes into which the integrin αv, the integrin α6, and the integrin β4 were introduced individually and exosomes into which the integrin α6 and the integrin β4 were introduced at the same time were incorporated into pulmonary fibroblasts. On the other hand, in exosomes in which the function of the integrin α or integrin β was inhibited using anti-integrin αv antibodies, anti-integrin α6 antibodies, or anti-integrin β4 antibodies, incorporation into pulmonary fibroblasts was reduced to about 50% to 60%. These facts indicate that, when at least the integrin α and/or integrin β was introduced into exosomes, the exosomes were more likely to be incorporated into pulmonary fibroblasts.

### <Experimental Example 3-4; comparison of incorporation of exosome into HPF cells and HUVEC cells>

HPF cells and human umbilical vein endothelial cells (HUVEC) were used as model target cells for examining incorporation into exosomes. HPF cells and exosomes were prepared in the same manner as in [Examination of cell incorporation of integrin α gene- or integrin β gene-introduced HEK293F cell-derived exosome into HPF cells] in Experimental Example 3-3. The method of preparing HUVEC cells is shown below.

### [Preparation of HUVEC cells]

HUVEC cells that had been subcultured in advance in an Endothelial Cell Growth Medium 2 (endothelial cell growth medium, commercially available from PromoCell) in a T75 flask were detached from the plate surface by a trypsin method and collected, and the number of cells was counted. The cells were seeded in a 24-well plate at 5×10⁴ cells/500 µL/well, and cultured for 2 days under conditions of 37°C and 5% CO₂.

### [Cell incorporation of integrin α gene- or integrin β gene-introduced HEK293F cell-derived exosome into HUVEC cells]

HEK293F cell-derived exosomes into which the above integrin α gene and/or integrin β gene was introduced were suspended in 100 µL of PBS. Green fluorescence staining was performed according to the protocol of ExoSparkler Exosome Membrane Labeling Kit-Green (commercially available from Dojindo Molecular Technologies, Inc.). HUVEC cells were washed with 200 µL of PBS twice, and 500 µL of a new Endothelial Cell Growth Medium 2 (endothelial cell growth medium, commercially available from PromoCell) was added. Fluorescently labeled exosomes were added to the medium, and culturing was performed for 4 hours under conditions of 37°C and 5% CO₂.

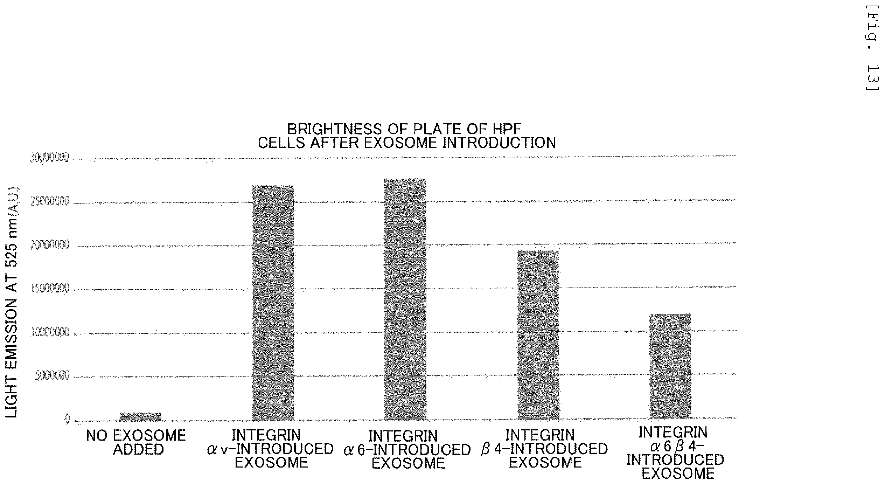

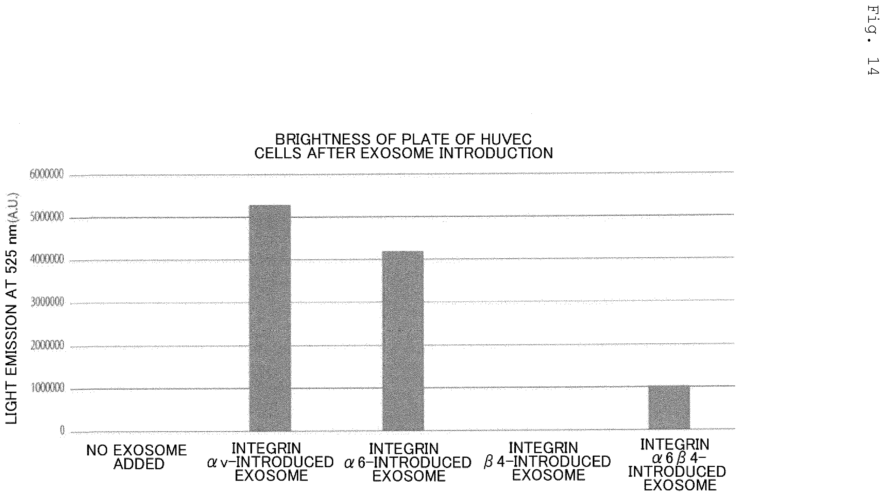

Using HPF cells and HUVEC cells, according to the above protocol and [Examination of cell incorporation of integrin α gene- or integrin β gene-introduced HEK293F cell-derived exosome into HPF cells] in Experimental Example 3-3, the exosome in which the integrin α6 and/or integrin β4 was expressed was added. The treated HPF cells and HUVEC cells were observed using an all-in-one fluorescence microscope BZ-X810 (commercially available from Keyence Corporation) at an excitation wavelength of 470/40 nm and a light emission wavelength of 525/50 nm, and the brightness (A.U.) of the plate was measured. The results are shown in Table 10, and Fig. 13 and Fig. 14.

**[Table 11]**

| Table 10: Brightness of plate of HPF cells and HUVEC cells after exosome introduction | | |
|---|---|---|
| | Brightness (HPF cells, A.U.) | Brightness (HUVEC cells, A.U.) |
| No exosome added | 8.9 × 10⁵ | 3.2 × 10³ |
| Integrin *α*v-introduced exosome | 2.7 × 10⁷ | 5.3 × 10⁶ |
| Integrin *α*6-introduced exosome | 2.8 × 10⁷ | 4.2 × 10⁶ |
| Integrin *β*4-introduced exosome | 1.9 × 10⁷ | 3.9 × 10³ |
| Integrin *α*6*β*4-introduced exosome | 1.2 × 10⁷ | 1.0 × 10⁶ |

As can be clearly understood from Table 10, and Fig. 13 and Fig. 14, the exosome into which the integrin α6 was introduced was specifically incorporated into both HPF cells and HUVEC cells. On the other hand, the exosome into which the integrin β4 was introduced was specifically incorporated into HPF cells in the presence and absence of integrin α, but was hardly incorporated into HUVEC cells.

### <Experimental Example 3-5; preparation of peptide-added hybrid liposome exosome>

As model target cells for examining incorporation into exosomes, HPF cells and HUVEC cells were used.

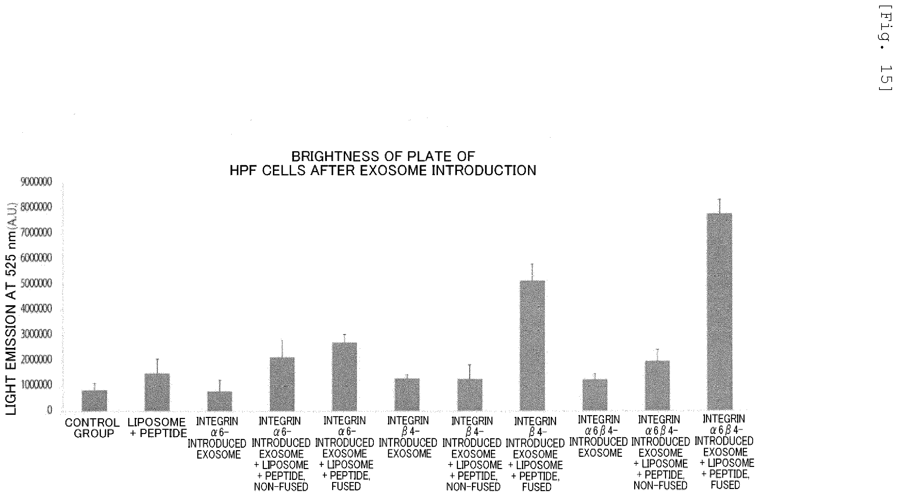

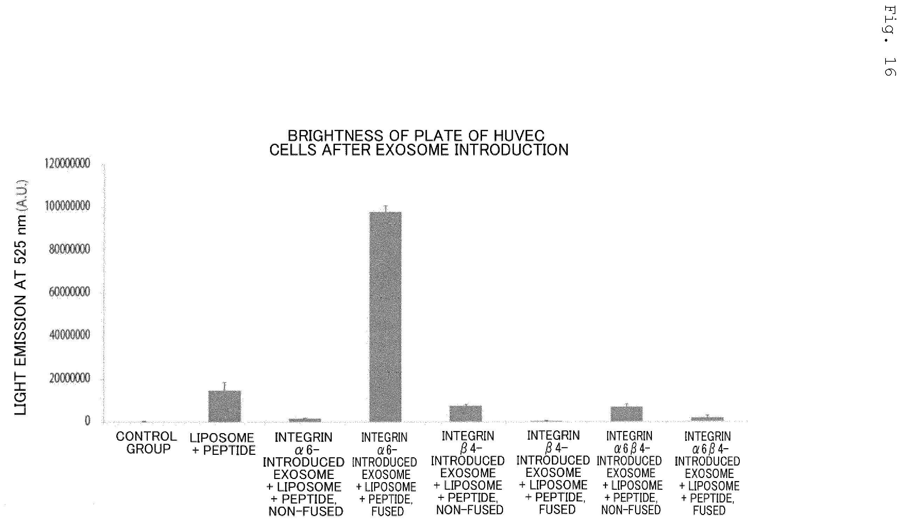

FD-U PL (1,2-dioleyl-sn-glycero-3-phosphocholine (DOPC):cholesterol=70:30) was weighed out in a 1.5 mL tube and suspended in PBS(-). Peptides fluorescently labeled with FITC were collected in a 1.5 mL tube and dissolved in PBS. This peptide solution and DSPE-PEG2000-NHS solution (DSPE; distearylphosphatidylethanolamine) were mixed and left at room temperature for 2 hours, and thus NHS was reacted with amino groups of the peptides. The mixed solution containing FD-U PL and DSPE-PEG2000-NHS-peptides was heated at 37°C for 30 minutes. Here, for the peptides, those obtained by binding FITC to the C-terminal of the peptide shown in SEQ ID No. 3 and those obtained by binding FIPTC to the C-terminal of the peptide shown in SEQ ID No. 5 were separately used. The peptide-added liposome and the exosome solution obtained from cells in which the integrin α6 or integrin β4 was overexpressed were mixed. In addition, a PEG-8000 solution was added so that the concentration was 30%. The mixture was left at 40°C for 2 hours, and the liposome and the exosome were fused. As a PEG solution control, a PBS(-) solution was used (indicated as "non-fused"). The obtained hybrid liposome exosome was subjected to an experiment of incorporating into HPF cells and HUVEC cells according to the protocol described in Experimental Example 3-3. The treated HPF cells and HUVEC cells were observed using a fluorescence microscope BZ-X810 (commercially available from Keyence Corporation) at an excitation wavelength of 470/40 nm and a light emission wavelength of 525/50 nm, and the brightness (A.U.) of the plate was measured. The results obtained when the peptide represented by SEQ ID No. 3 was used as representative data are shown in Table 11 and Table 12, and Fig. 15 and Fig. 16. Here, when the peptide of SEQ ID No. 5 was used in place of the peptide of SEQ ID No. 3, generally, the results showed similar trends to those shown in Table 11 and Table 12, and Fig. 15 and Fig. 16.

**[Table 12]**

| Table 11: Brightness of plate of HPF cells after exosome introduction | | |
|---|---|---|
| | Number of data items | Brightness (average, A.U.) |
| Control group | 4 | 8.3 × 10⁵ |
| Liposome + peptide | 4 | 1.5 × 10⁶ |
| Integrin *α*6-introduced exosome | 4 | 7.8 × 10⁵ |
| Integrin *α*6-introduced exosome + liposome + peptide, non-fused | 4 | 2.1 × 10⁶ |
| Integrin *α*6-introduced exosome + liposome + peptide, fused | 4 | 2.7 × 10⁶ |
| Integrin *β*4-introduced exosome | 4 | 1.3 × 10⁶ |
| Integrin *β*4-introduced exosome + liposome + peptide, non-fused | 4 | 1.2 × 10⁶ |
| Integrin *β*4-introduced exosome + liposome + peptide, fused | 4 | 5.1 × 10⁶ |
| Integrin *α*6*β*4-introduced exosome | 4 | 1.2 × 10⁶ |
| Integrin *α*6*β*4-introduced exosome + liposome + peptide, non-fused | 4 | 2.0 × 10⁶ |
| Integrin *α*6*β*4-introduced exosome + liposome + peptide, fused | 4 | 7.7 × 10⁶ |

**[Table 13]**

| Table 12: Brightness of plate of HUVEC cells after exosome introduction | | |
|---|---|---|
| | Number of data items | Brightness (average, A.U.) |
| Control group | 4 | 1.7 × 10⁵ |
| Liposome + peptide | 4 | 1.5 × 10⁷ |
| Integrin *α*6-introduced exosome + liposome + peptide, non-fused | 4 | 1.5 × 10⁶ |
| Integrin *α*6-introduced exosome + liposome + peptide, fused | 4 | 9.8 × 10⁷ |
| Integrin *β*4-introduced exosome + liposome + peptide, non-fused | 4 | 7.4 × 10⁶ |
| Integrin *β*4-introduced exosome + liposome + peptide, fused | 4 | 3.5 × 10⁵ |
| Integrin *α*6*β*4-introduced exosome + liposome + peptide, non-fused | 4 | 6.9 × 10⁶ |
| Integrin *α*6*β*4-introduced exosome + liposome + peptide, fused | 4 | 1.8 × 10⁶ |

As can be clearly understood from Table 11 and Fig. 15, the hybrid liposome exosome into which the integrin β4 was introduced was incorporated into HPF cells with strong targeting, but the hybrid liposome exosome into which both the integrin α6 and the integrin β6 were introduced showed very strong targeting to HPF cells. On the other hand, as can be clearly understood from Table 12 and Fig. 16, the hybrid liposome exosome into which the integrin β4 was introduced was hardly incorporated into HUVEC cells regardless of the presence and absence of integrin α6. Such highly specific strong targeting to HPF cells makes the hybrid liposome exosome-containing composition, which contained the hybrid liposome exosome of the present embodiment, extremely useful for delivering an active component for the treatment of lung diseases.

In addition, as can be clearly understood from Table 11 and Fig. 15, when the exosome into which the integrin α6 and the integrin β4 were introduced was fused with the liposome retaining peptides, the result of incorporation into HPF cells was improved by 290% or more, compared to the mixture of corresponding non-fused exosomes and liposomes.

### <Experimental Example 4-1; proteome analysis in integrin-overexpressing exosome>

The obtained exosomes were separated from integrin non-overexpression HEK293F cells as a control group and HEK293F cells prepared in Experimental Example 1-1, and Experimental Example 1-2 in which the integrin α6 or integrin β4 was overexpressed. The total protein concentration and the total protein amount of samples were 26.5 µg/mL and 19.9 µg for the exosomes obtained from integrin non-overexpression HEK293F cells, 23.8 µg/mL and 17.9 µg for the exosomes obtained from integrin α6 overexpression cells, and 29.1 µg/mL and 21.8 µg for and the exosomes obtained from integrin β4 overexpression cells. These samples were stored at -80°C until the analysis operation started.

### [Separation of protein]

These samples were subjected to an acetone precipitation treatment and thus protein fractions were separated. Specifically, 375 µL of each of the above samples was divided into three equal parts and 125 µL of the sample was collected in three new containers. Subsequently, cold acetone in an amount 8 times the volume of the suspension was added to each container, and the mixture was left overnight under conditions of -20°C. The mixture was centrifuged (15,000×g, 4°C, for 15 minutes), the supernatant was removed, and the precipitate was re-suspended in cold acetone. The suspension was mixed with inversion and centrifuged again (15,000×g, 4°C, for 5 minutes), the supernatant was removed again, and finally the precipitate was air-dried (left at room temperature for 30 minutes). The dried precipitates from three containers were combined and dissolved in 20 µL of a solubilization buffer solution to obtain a protein dissolution solution. Here, the composition of the solubilization buffer solution was 8 M urea, 50 mM Tris-HCl, pH 8.0.

### [Fragmentation of protein]

As a pretreatment for LC-MS/MS, the total amount of the protein dissolution solution was subjected to hydrolysis with trypsin, and proteins were fragmented. 2.2 µL of a 100 mM dithiothreitol (DTT) solution was added to the protein dissolution solution, the mixture was incubated at 37°C for 30 minutes (final concentration of DTT: 10 mM), and 2.5 µL of a 250 mM iodoacetamide (IAA) solution was then added (final concentration of IAA: 25 mM) and the mixture was left in a dark place for 20 minutes to perform reductive alkylation. After the reductive alkylation treatment, 55.3 µL of 50 mM Tris-HCl (pH 8.0) was added to lower the urea concentration of the solution to 2 M. Finally, 1 µL of a 1 µg/µL trypsin solution was added, the mixture was incubated at 37°C for 16 hours to perform a hydrolysis reaction (final concentration of trypsin: 12.3 ng/µL). The peptide solution after the decomposition reaction was desalted using C18 STAGE Tip (Rappsilber, J. et al., Anal. Chem. 2003, 75:663-70). The sample after the desalting treatment was dried under a reduced pressure.

### [LC-MS/MS]

The dried peptide sample was dissolved in 10 µL of a solvent composed of water, acetonitrile and trifluoroacetic acid (a volume ratio of 98:2:0.1), and 7 µL thereof was subjected to LC-MS/MS under the following setting conditions and treatment conditions.

### (LC: Ultimate 3000 RSLCnano (commercially available from Thermo Fisher Scientific))

·C18 column for analysis (Tip column): Nano HPLC Capillary Column (a particle size of 3 µm, an inner diameter of 75 µm, a length of 15 cm, commercially available from Nikkyo Technos, Co., Ltd.)
·composition of mobile phase A:[water]:[formic acid]=100:0.1 (volume ratio)
·composition of mobile phase B:[water]:[acetonitrile]:[formic acid]=10:90:0.1 (volume ratio)·acetonitrile feeding gradient (min, %B, %acetonitrile):(0,5,4.5)→(5,5,4.5)→(120,40,36)→(120.01,95,85.5)→(130,95,85.5) →(130.01,5,4.5)→(145,5,4.5)·flow rate: 350 nL/min

### (MS/MS: Q Exactive Orbitrap mass spectrometer (commercially available from Thermo Fisher Scientific))

·ion mode: positive ion mode
·set temperature of ion transfer capillary: 250°C
·FullScan m/z scanning range: 310-1500
·mass resolution: 70,000(MS), 17,500 (MS/MS)
·Lock Mass: On (Reference m/z=391.28429, 445.12003)
·data acquisition method: "Top 10 method"

That is, among ions detected by each FullScan MS (m/z 310-1500), measurement variables were set to acquire MS/MS data in descending order of 10 high-intensity ions. In this case, ions with valences 2, 3 and 4 were selection targets.

### (Peak intensity information output and normalization process, and amino acid sequence database search)

The measure data obtained from each sample by LC-MS/MS was processed according to the following procedure. That is, using software Proteome Discoverer (ver. 2.2) (hereinafter referred to as "PD2.2," commercially available from Thermo Fisher Scientific), peak alignment of each type of LC-MS/MS data and sequence database search were performed. For sequence database search, Mascot software (commercially available from Matrix Science) was used via PD2.2. Identification information of a peptide considered significant was linked to each detection peak, and the intensity value of the linked detection peak was used as the detection intensity of the peptide. Here, the normalization process of the peak detection intensity was performed according to the PD2.2 algorithm.

The amino acid sequence database used for searching for amino acid sequences was constructed by combining the following two databases.
·human-derived Swiss-Prot protein entries registered in UniProt 2020_2nd edition (a total of 20,380 entries)
·among amino acid sequences of protein contaminants acquired from The Global Proteome Machine Organization, amino acid sequences derived from non-humans (a total of 48)

Search conditions for searching for amino acid sequences in the sequence database were as follows.
·Enzyme, Trypsin
·Maximum missed cleavage, 2
·Peptide tolerance, ±5ppm
·MS/MS tolerance, ±0.02Da
·Mass, monoisotopic mass
·Fixed Modification, Carbamidomethyl (C, +57.021Da)
·Variable Modification:Oxidation (M, +15.995)

The significance test in the peptide identification was performed using False discovery rate (FDR) as an index, and the score threshold for peptide identification was adjusted so that FDR was 1%. The PSM score was adjusted using "Target Decoy PSM validator" in PD2.2.

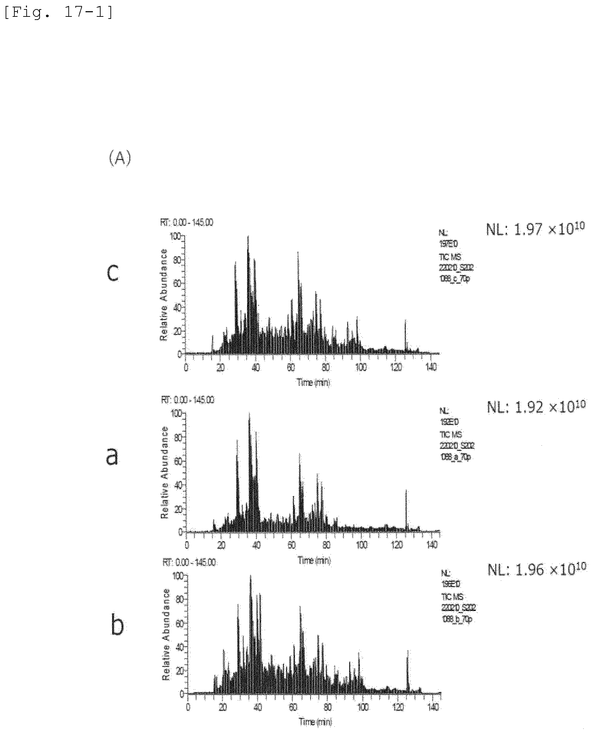

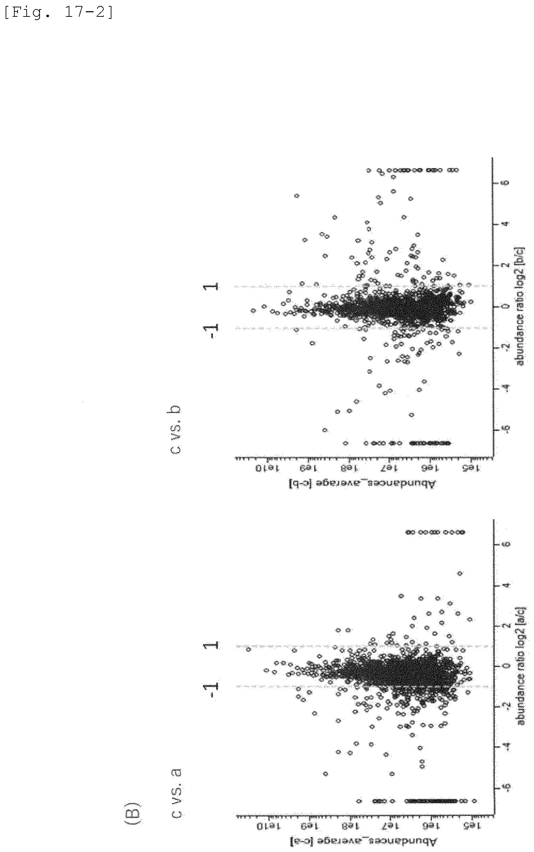

Some of the results are shown in Fig. 17. Here, in Fig. 17(B), the horizontal axis represents the protein amount between two samples as a multiple value (expressed as a logarithm with a base of 2), and the vertical axis represents the average of protein amounts in two samples, indicating the relative abundance of target proteins in all proteins (expressed as a logarithm with a base of 10). When the multiple value was a value of -6.64 (1/100) or less and 6.64 (100 times) or more, it was set as -6.64 and 6.64. In Fig. 17(B), plots that are far from the center in the horizontal direction indicate that the amount of transfer to exosomes varies greatly due to integrin overexpression, and a higher value on the vertical axis indicates a higher expression level. As can be clearly understood from Fig. 17, it was found that the protein disulfide isomerase A4 (PDIA4), which is a type of protein disulfide isomerase, was found in exosomes even if the integrin α4 was overexpressed, and particularly, when the integrin β6 was overexpressed, a large amount of the protein disulfide isomerase was transferred to the exosomes.

### <Experimental Example 4-2; examination of targeting of PDIA4 knockdown HEK293 cell-derived exosome>

### [Preparation of HEK293F cells for gene introduction]

HEK293F cells that had been subcultured and maintained in advance in an HE100 medium (glutamine, penicillin-streptomycin solution mixture) (GMEP) in a 100 mm culture dish were removed from the culture surface by pipetting, and collected, and the number of cells was counted. The cells were seeded in a 6-well CellBIND (commercially available from Corning) at 2×10⁵ cells/3 ml/well and cultured overnight under conditions of 37°C and 5% CO₂.

### [Introduction of integrin gene into HEK293F cells]

According to the method described in Experimental Example 1-2, the integrin gene-carrying DNA vector was introduced into HEK293F cells. In this case, the final concentration of DNA added to the HEK293F cell suspension was adjusted to be 2.5 µg DNA/well.

### [Introduction of PDIA4 siRNA into HEK293F cells]

After Integrin genes were introduced into HEK293F cells, the cells were washed twice with 2 ml of PBS. siRNA targeting PDIA4 (MISSION (registered trademark) esiRNA (commercially available from Sigma Aldrich) was introduced according to the protocol of MISSION (registered trademark) siRNA Transfection Reagent (commercially available from Sigma Aldrich). 10 µL of siRNA Transfection Reagent was used per well, and the concentration of siRNA was adjusted to be 10 nM.

### [Collection of HEK293F cell-derived exosome]

The supernatant was removed, and the medium was replaced with 5 ml of an HE100 medium. Culturing was performed for 16 days, and the supernatant was collected. Centrifugation was performed at 3,000 g×30 min at room temperature, and the supernatant was collected into a new 15 mL tube. The sample was stored at -80°C until the exosomes were collected. The exosomes were collected from the centrifuged supernatant using the MagCapture method.

### [Flow cytometry]

A sample for flow cytometry was prepared according to the protocol of the PS Capture Exosome flow cytometry kit. In addition, Attune (registered trademark) Acoustic Focusing Flow Cytometer (commercially available from Thermo Fisher Scientific) was used as the flow cytometer. PE-Cy7 anti-human CD63 mouse antibodies, and clone H5C6(RUO) were used as antibodies for exosome detection, and ERp72 polyclonal antibodies, Alexa Fluor 488 (commercially available from Thermo Fisher Scientific) were used for PDIA4 detection.

### [Examination of incorporating integrin gene-introduced HEK293F-derived exosome into HPF cells]

HPF cells that had been subcultured and maintained in advance in a fibroblast growth medium 2 (model number: D12042, commercially available from PromoCell) in a T75 flask were removed from the culture surface by a trypsin method and collected, and the number of cells was counted. The cells were seeded in a 24-well at 5×10⁴ cells/500 µl/well, and cultured overnight under conditions of 37°C and 5% CO₂.

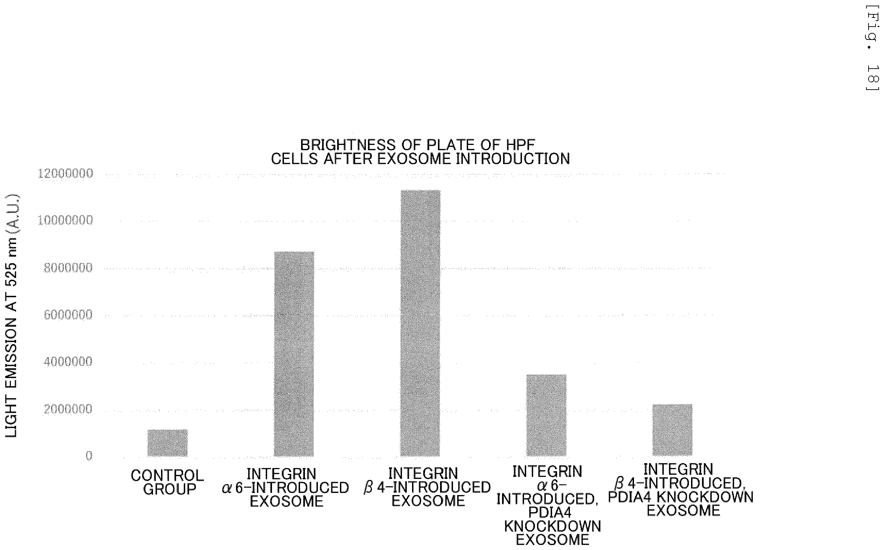

HEK293F-derived exosomes collected by the MagCapture method were suspended in 100 µL of PBS. Green fluorescence staining was performed according to the protocol of ExoSparkler Exosome Membrane Labeling Kit-Green (commercially available from Dojindo Laboratories). HUVEC cells were washed with 200 µL of PBS twice, and 500 µL of a new Endothelial Cell Growth Medium 2 (endothelial cell growth medium, commercially available from PromoCell) was added. Fluorescently labeled exosomes were added to the medium, and culturing was performed for 4 hours under conditions of 37°C and 5% CO₂. After the sample medium was removed, washing with 200 µL of PBS was performed twice, HEK293F cell-derived exosomes in the supernatant were removed and observed under a fluorescence microscope, and the percentage of HPF cells incorporating exosomes was measured. The results are shown in Table 13 and Fig. 18.

**[Table 14]**

| Table 13: Brightness of plate of HPF cells after exosome introduction | |
|---|---|
| | Brightness (average, A.U.) |
| Control group | 1.1 × 10⁶ |
| Integrin *α*6-introduced exosome | 8.7 × 10⁶ |
| Integrin *β*4-introduced exosome | 1.1 × 10⁷ |
| Integrin *α*6-introduced, PDIA4 knockdown exosome | 3.5 × 10⁶ |
| Integrin *β*4-introduced, PDIA4 knockdown exosome | 2.2 × 10⁶ |

As can be clearly understood from Table 13 and Fig. 18, the integrin α-introduced exosome and the integrin β-introduced exosome obtained from the PDIA4 knockdown cells had a statistically significant decrease in the frequency of transfer to HPF cells. Therefore, it was found that the protein disulfide isomerase (PDI) was highly involved in strong targeting to cells according to introduction of the integrin α and/or integrin β.

## Claims

1. An exosome comprising at least one exosome marker protein selected from among CD63, CD81 and CD9, and an integrin α and/or an integrin β in a lipid bilayer.

2. The exosome according to claim 1, further comprising a protein disulfide isomerase (PDI) in the lipid bilayer.

3. The exosome according to claim 2,
wherein the exosome marker protein is selected from among CD63, CD81 and CD9, and
wherein the exosome contains 0.01 ng or more and 100 ng or less of the protein disulfide isomerase (PDI) per 1 ng of the total amount of the exosome marker protein present in the exosome.

4. The exosome according to claim 2 or 3,
wherein the molar ratio of the protein disulfide isomerase (PDI) to the integrin α and the integrin β is 1:0.1 to 1:10.

5. The exosome according to claim 2 or 3,
wherein the protein disulfide isomerase (PDI) is selected from among
(i) human protein disulfide isomerase A4 including an amino acid sequence shown in SEQ ID No. 6;
(ii) human protein disulfide isomerase A1 including an amino acid sequence shown in SEQ ID No. 7;
(iii) human protein disulfide isomerase A3 including an amino acid sequence shown in SEQ ID No. 8;
(iv) human protein disulfide isomerase A6 including an amino acid sequence shown in SEQ ID No. 9; and
(v) a mutant protein including an amino acid sequence having at least 90% sequence homology to the amino acid sequence shown by any of SEQ ID Nos. 6 to 9 and functioning as a protein disulfide isomerase.

6. The exosome according to claim 1 or 2,
wherein the exosome marker protein is selected from among CD63, CD81 and CD9, and
wherein the exosome contains 0.01 ng or more and 100 ng or less of the integrin α per 1 ng of the total amount of the exosome marker protein present in the exosome, or
0.01 ng or more and 100 or less of the integrin β per 1 ng of the total amount of the exosome marker protein present in the exosome.

7. The exosome according to claim 1 or 2,
wherein the integrin α is selected from among
(i) human integrin α4 including an amino acid sequence shown in SEQ ID No. 10;
(ii) human integrin αv including an amino acid sequence shown in SEQ ID No. 11;
(iii) human integrin α6 including an amino acid sequence shown in SEQ ID No. 12; and
(iv) a mutant protein including an amino acid sequence having at least 90% sequence homology to the amino acid sequence shown by any of SEQ ID Nos. 10 to 12 and functioning as an integrin α, and
wherein the integrin β is selected from among
(v) human integrin β4 including an amino acid sequence shown in SEQ ID No. 13;
(vi) human integrin β5 including an amino acid sequence shown in SEQ ID No. 14;
(vii) human integrin β6 including an amino acid sequence shown in SEQ ID No. 15; and
(viii) a mutant protein including an amino acid sequence having at least 90% sequence homology to the amino acid sequence shown by any of SEQ ID Nos. 13 to 15 and functioning as an integrin β.

8. A method of forming an exosome, the method comprising:
an overexpression step in which an integrin α and/or an integrin β is overexpressed in cells; and
a collection step in which the exosome according to claim 1 or 2 is collected from the cells in which the integrin α and/or the integrin β is overexpressed.

9. The method of forming an exosome according to claim 8,
wherein the cells are at least one selected from the group consisting of pluripotent stem cells, mesenchymal stem cells, ectoderm-derived cells, mesoderm-derived cells, and endoderm-derived cells.

10. A method of forming a hybrid liposome exosome, the method comprising
a hybrid liposome exosome forming step in which the exosome formed by the method of forming an exosome according to claim 8 or 9 is fused with a liposome to form a hybrid liposome exosome.

11. A hybrid liposome exosome formed by the method of forming a hybrid liposome exosome according to claim 10.

12. A hybrid liposome exosome comprising at least one exosome marker protein selected from among CD63, CD81 and CD9, an integrin α, and an integrin β in a lipid bilayer.

13. The hybrid liposome exosome according to claim 12, further comprising a protein disulfide isomerase (PDI) in the lipid bilayer.

14. The hybrid liposome exosome according to claim 13,
wherein the molar ratio of the protein disulfide isomerase (PDI) to the integrin α and the integrin β is 1:0.1 to 1:10.

15. The hybrid liposome exosome according to claim 13 or 14,
wherein the protein disulfide isomerase (PDI) is selected from among
(i) human protein disulfide isomerase A4 including an amino acid sequence shown in SEQ ID No. 6;
(ii) human protein disulfide isomerase A1 including an amino acid sequence shown in SEQ ID No. 7;
(iii) human protein disulfide isomerase A3 including an amino acid sequence shown in SEQ ID No. 8;
(iv) human protein disulfide isomerase A6 including an amino acid sequence shown in SEQ ID No. 9; and
(v) a mutant protein including an amino acid sequence having at least 90% sequence homology to the amino acid sequence shown by any of SEQ ID Nos. 6 to 9 and functioning as a protein disulfide isomerase.

16. The hybrid liposome exosome according to claim 12 or 13,
wherein the integrin α is selected from among
(i) human integrin α4 including an amino acid sequence shown in SEQ ID No. 10;
(ii) human integrin αv including an amino acid sequence shown in SEQ ID No. 11;
(iii) human integrin α6 including an amino acid sequence shown in SEQ ID No. 12; and
(iv) a mutant protein including an amino acid sequence having at least 90% sequence homology to the amino acid sequence shown by any of SEQ ID Nos. 10 to 12 and functioning as an integrin α, and
wherein the integrin β is selected from among
(v) human integrin β4 including an amino acid sequence shown in SEQ ID No. 13;
(vi) human integrin β5 including an amino acid sequence shown in SEQ ID No. 14;
(vii) human integrin β6 including an amino acid sequence shown in SEQ ID No. 15; and
(viii) a mutant protein including an amino acid sequence having at least 90% sequence homology to the amino acid sequence shown by any of SEQ ID Nos. 13 to 15 and functioning as an integrin β.

17. A composition comprising the exosome according to claim 1 or the hybrid liposome exosome according to claim 11 or 12, and a pharmaceutically acceptable carrier.

18. The composition according to claim 17, which is used to treat a lung disease, alleviate lung disease symptoms, and/or prevent a lung disease.
